(19) Europäisches Patentamt
European Patent Office
Office européen des brevets

(11) EP 2 328 546 B1

(12) # EUROPEAN PATENT SPECIFICATION

(45) Date of publication and mention of the grant of the patent:
**22.11.2017 Bulletin 2017/47**

(21) Application number: **09817926.0**

(22) Date of filing: **30.09.2009**

(51) Int Cl.:
***A61K 8/68*** *(2006.01)* ***A61K 8/36*** *(2006.01)*
***A61Q 19/00*** *(2006.01)* ***A61K 8/06*** *(2006.01)*

(86) International application number:
**PCT/JP2009/067439**

(87) International publication number:
**WO 2010/038899 (08.04.2010 Gazette 2010/14)**

(54) **AQUEOUS COSMETIC PREPARATION AND METHOD FOR PRODUCING THE SAME**

WÄSSRIGES KOSMETIKPRÄPARAT UND VERFAHREN ZU DESSEN HERSTELLUNG

PRÉPARATION COSMÉTIQUE AQUEUSE ET PROCÉDÉ DE PRODUCTION ASSOCIÉ

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **30.09.2008 JP 2008254538**

(43) Date of publication of application:
**08.06.2011 Bulletin 2011/23**

(73) Proprietor: **FUJIFILM Corporation**
**Tokyo 106-8620 (JP)**

(72) Inventors:
- **TASHIRO, Tomoko**
  **Ashigarakami-gun**
  **Kanagawa 258-8577 (JP)**
- **MORI, Hisahiro**
  **Ashigarakami-gun**
  **Kanagawa 258-8577 (JP)**
- **SERIZAWA, Shinichiro**
  **Ashigarakami-gun**
  **Kanagawa 258-8577 (JP)**
- **NAKAMURA, Yoshisada**
  **Ashigarakami-gun**
  **Kanagawa 258-8577 (JP)**

(74) Representative: **Klunker IP**
**Patentanwälte PartG mbB**
**Destouchesstraße 68**
**80796 München (DE)**

(56) References cited:
**EP-A1- 0 885 896** **JP-A- 2005 002 018**
**JP-T- 2007 516 067** **US-A1- 2006 057 091**

- **DATABASE WPI Week 200507 Thomson Scientific, London, GB; AN 2005-061577 XP002735849, & JP 2005 002070 A (QP CORP) 6 January 2005 (2005-01-06)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 328 546 B1

## Description

Technical Field

The present invention relates to an aqueous cosmetic preparation and a method for producing the aqueous cosmetic preparation.

Background Art

Ceramide is present in a stratum corneum of the skin and constructs a lipid barrier necessary for retaining water, and thereby it plays an important role for maintaining moisture. Ceramide in a stratum corneum is produced by degradation of cerebroside with an enzyme called cerebrosidase. It is known that a part of ceramide is changed into phytosphingosine and sphingosine with an enzyme called ceramidase, and they are important as an agent of regulating proliferation and differentiation of cells. In a human skin, seven kinds of ceramides are present, and have different functions, respectively.

However, since ceramide is a substance having high crystallizability, has low solubility in other oil solution, and precipitates a crystal at a low temperature, it was difficult to maintain stability when incorporated into cosmetics. Further, an aqueous ceramide dispersion may be dispersed using surfactants, but it is difficult to make a particle diameter of an dispersion sufficiently small, and thereby the dispersion inferior in transparency may be produced in some cases.

An emulsifying composition that contains a specific group of sphingoglycolipid having moisturing effect, preventing effect on chapped skin, and emulsifying effect is disclosed as a composition containing ceramides (for example, refer to Japanese Patent Application Laid-Open (JP-A) No. 2000-51676).

A cosmetic additive combined with ceramide which contains cholesterol, fatty acid, and water-soluble polymer (for example, refer to JP-A No. 7-187987) is disclosed. As a composition for external use which is excellent in stability under rapidly changing temperature conditions and has good after-use feel, a water-in-oil type emulsifying composition obtained by using sphingosines and salt formed with a specific fatty acid as an emulsifying agent and adding an oil-soluble antioxidant at a specific ratio (for example, refer to JP-A No. 2006-335692) is disclosed.

As pharmaceutical preparation technique, a method for producing an additive agent for cosmetic in which a crude dispersion solution of sphingoglycolipid is microparticulated using a specified jet flow in order to sufficiently exhibit the emollient effect of sphingoglycolipid is disclosed (for example, refer to JP-ANo. 11-310512).

On the other hand, a process for blending specific fatty acids and specific surfactants is disclosed as a technique for transparently solubilizing and stably blending ceramides (for example, refer to JP-A Nos. 2001-139796 and 2001-316217). However, since the ceramide has high crystallinity as described above, even if a stable water-emulsified dispersion is produced, it becomes cloudy or precipitates are generated in many cases when it is mixed with a general cosmetic component. When the ceramide is stabilized by particularly fatty acid, only a limited number of cosmetic components can be blended together. Thus, the final blending concentration in the cosmetic has to be reduced.

With respect to the blending of fatty acid and ceramides with cosmetics, at present, guidelines for a technique/formulation for blending particles containing ceramides (in a state in which the particle diameter is small) into a cosmetic at high concentration have not yet been provided.

On the other hand, a process for blending specific fatty acids and specific surfactants is disclosed as a technique for transparently solbilizing ceramides and stably blending (for example, refer to JP-A Nos. 2001-139796 and 2001-316217). However, the ceramide has a high crystallinity as described above, therefore, even if stable water-emulsified dispersion is produced, it becomes turbid or precipitates are generated in many cases when it is mixed with a general cosmetic component. When the ceramide is stabilized by particularly fatty acid, the cosmetic component to be blended together is limited. Thus, the final compounding concentration to the cosmetic has to be reduced.

With reference to the case where fatty acid and ceramides are blended with cosmetics, at the present, the guideline for the technique/formulation for blending particles containing ceramides (in a state where the particle diameter is small) into the cosmetic at high concentrations has not been provided yet.

DISCLOSURE OF INVENTION

The present invention has been made in view of the above-described circumstances. It provides an aqueous cosmetic preparation in which a ceramide analog-containing particle with a very small particle diameter is stably dispersed and which has excellent stability over time (temporal stability), as well as a method for producing the same.

According to a first aspect of the invention, there is provided an aqueous cosmetic preparation which includes a ceramide analog-containing particle that contains at least a ceramide analog and has a volume average particle diameter of from 2 nm to 150 nm, the particle being dispersed in an aqueous phase as an oil-phase component; and a fatty acid having 10 to 30 carbon atoms or a salt thereof, the aqueous cosmetic preparation satisfying both conditions

that the pH is 6.5 or more and that the electric conductivity is 5.0 mS/cm or less.

According to a second aspect of the invention, the aqueous cosmetic preparation of the first aspect is provided in which the pH and the electric conductivity (mS/cm) satisfy a relationship expressed by the following Equation (A).

$$\text{Electric conductivity (mS/cm)} \leq 2.2 \times \text{pH} - 7 \qquad \text{[Equation (A)]}$$

According to a fourth aspect of the invention, the aqueous cosmetic preparation of any one of the first to third aspects is provided in which the volume average particle diameter of the particle is from 5 nm to 100 nm.

According to a fifth aspect of the invention, the aqueous cosmetic preparation of any one of the first to fourth aspects further includes polyhydric alcohol.

According to a sixth aspect of the invention, the aqueous cosmetic preparation of any one of the first to fifth aspects further includes a polymeric compound.

According to a seventh aspect of the invention, the aqueous cosmetic preparation of any one of the first to sixth aspects is provided in which the fatty acid having 10 to 30 carbon atoms is in liquid form at 30°C.

According to an eighth aspect of the invention, the aqueous cosmetic preparation of any one of the first to seventh aspects is provided in which the fatty acid having 10 to 30 carbon atoms or a salt thereof is at least one compound selected from the group consisting of lauric acid, isostearic acid, oleic acid, $\gamma$-linolenic acid, $\alpha$-linolenic acid, and respective salts thereof.

According to a ninth aspect of the invention, there is provided a method for producing the aqueous cosmetic preparation of any one of the first to eighth aspects of the invention which includes: preparing a ceramide dispersion by mixing an oil phase component which contains at least a ceramide analog and an aqueous phase component at 40°C or less; and mixing the ceramide dispersion and an aqueous composition.

According to a tenth aspect of the invention, the method for producing the aqueous cosmetic preparation of ninth aspect further includes dissolving the ceramide analog.

According to an eleventh aspect of the invention, the method for producing the aqueous cosmetic preparation of the tenth aspect is provided in which the good solvent of the ceramide analog is a water-soluble organic solvent.

According to a twelfth aspect of the invention, the method for producing the aqueous cosmetic preparation of any one of the ninth to eleventh aspects is provided in which the oil phase component and the aqueous phase component are independently passed through a micro path having a cross-section area at the narrowest portion thereof of from 1 $\mu m^2$ to 1 $mm^2$, whereafter the oil phase component and the aqueous phase component are combined and mixed.

BRIEF DESCRIPTION OF THE DRAWINGS

Fig. 1 is an exploded perspective view of a micro device as one example of a micro mixer;
Fig. 2 is a schematic cross-sectional view of a T-shaped microreactor showing one example of mixing mechanism with a T-shaped microreactor;
Fig. 3 is a concept view a T-shaped microreactor showing one example of mixing mechanism with a T-shaped microreactor; and
Fig. 4 is a diagram in which the pH of the aqueous cosmetic preparations of Examples 1 to 10 and Comparative example 1 are plotted along the horizontal axis, and the electric conductivity (mS/cm) of the aqueous cosmetic preparations are plotted along the vertical axis.

DESCRIPTION OF THE PREFERRED EMBODIMENT

1. Aqueous cosmetic preparation

The aqueous cosmetic preparation of the present invention includes at least a ceramide analog-containing particle that contains at least a ceramide analog and has a volume average particle diameter of from 2 nm to 150 nm, and the particle is dispersed in an aqueous phase as an oil-phase component; and a fatty acid having 10 to 30 carbon atoms or a salt thereof; and the aqueous cosmetic preparation satisfies at least either a condition where the pH is 6.5 or more or a condition where the electric conductivity is 5.0 mS/cm or less.

The pH and the electric conductivity in the aqueous cosmetic preparation of the invention preferably satisfy the relation of the following Equation (A), more preferably satisfy the relation of the following Equation (B), and further preferably satisfy the relation of the following Equation (C).

$$\text{Electric conductivity (mS/cm)} \leq 2.2 \times \text{pH} - 7 \quad \text{Equation (A)}$$

$$\text{Electric conductivity (mS/cm)} \leq 2.2 \times \text{pH} - 9 \quad \text{Equation (B)}$$

$$\text{Electric conductivity (mS/cm)} \leq 2.2 \times \text{pH} - 11 \quad \text{Equation (C)}$$

In the aqueous cosmetic preparation of the invention having these characteristics, particles containing ceramides are stably dispersed in a system and a good temporal stability may be exhibited.

The temporal stability of an aqueous cosmetic preparation which contains a ceramide analog-containing particle and a fatty acid component as essential ingredients, such as the aqueous cosmetic preparation of the invention, may be impaired when the pH of the system is low. This is thought to be because the fatty acid component contained in the aqueous cosmetic preparation functions as a dispersing agent of the ceramide analog-containing particle and exhibits excellent dispersive properties; however, the dispersibility and stability of the ceramide analog-containing particle are impaired by an increase in the fatty acid component included in the system which is in an undissociated state (-COOH) when the pH is low. Therefore, when the electrolytic concentration is high in a region of low pH, the stability tends to be further deteriorated. Thus, it is necessary to keep the salt concentration in the cosmetic as low as possible.

On the other hand, the fatty acid component included in the aqueous cosmetic preparation of the invention which is in an undissociated state ($-COO^-$) is increased in a region of high pH. For that reason, the electrostatic repulsion of the ceramide analog-containing particle is increased and the dispersion stability tends to be improved. Therefore, when the pH of the aqueous cosmetic preparation is in the high region, the dispersibility and stability of the ceramide analog-containing particle may be expected even if the salt concentration in the aqueous cosmetic preparation is high to a certain extent. Thus, it may be said that the pH and electrolytic concentration of the aqueous cosmetic preparation which contains the ceramide analog-containing particle and the fatty acid component greatly contributes to the dispersibility and stability of the ceramide analog-containing particle.

With reference to the electrolyte contained in the aqueous cosmetic preparation, the electric conductivity of the cosmetic becomes high when the content of the electrolyte is high.

Under such circumstances, the present inventors have conducted research focusing on the pH and electric conductivity in the aqueous cosmetic preparation containing the ceramide analog-containing particle and the fatty acid component. As a result, they have found that a specific pH and electric conductivity in the invention are correlated with the dispersibility of the ceramide analog-containing particle and the stability thereof, and have obtained a aqueous cosmetic preparation in which the ceramide analog-containing particle is

The pH and the electric conductivity which have the above-described relation in the aqueous cosmetic preparation of the invention will be further described.

When the pH is 6.5 or more, the generation of precipitates and suspended matter in the cosmetic and the generation of turbidity which is caused by coarsening due to aggregation of the ceramide analog-containing particle, is more effectively suppressed, which is preferable. The upper limit of the pH is preferably 10.0 or less, more preferably 9.0 or less, and most preferably 8.5 or less from the viewpoint of reduction of stimulation when the cosmetic is applied to the skin.

The pH of the aqueous cosmetic preparation of the invention may be adjusted by a base or acid. When the pH of the aqueous cosmetic preparation is adjusted, it may be set by adjusting the amount of acids such as hydrochloric acid, citric acid, lactic acid, or glutamic acid which are used for normal cosmetics and bases such as sodium hydroxide or arginine. The pH in the invention is a value measured by the glass electrode method.

When the aqueous cosmetic preparation of the invention shows an electric conductivity (mS/cm) of 5.0 mS/cm or less, the electric conductivity is more preferably 3.0 mS/cm or less, and most preferably 2.0 mS/cm or less. When the electric conductivity is 5.0 mS/cm or less, the generation of precipitates and suspended matter in the cosmetic preparation and the generation of turbidity which is caused by coarsening due to aggregation of the ceramide analog-containing particle, is more effectively suppressed, which is preferable.

The electric conductivity in the invention is a value measured by the AC two-electrode method.

In this regard, the aqueous cosmetic preparation of the invention has a configuration in which the ceramide analog-containing particle is dispersed as an oil phase component in the aqueous phase. Here, the ceramide analog-containing particle in the invention which is contained as the oil phase component may be in liquid or solid form at room temperature or may be in a state dissolved or dispersed in other oily components as long as the particle diameter of the particle is within the range specified by the invention.

As the aqueous phase, which is a dispersion medium in which the ceramide analog-containing particle is

dispersed, an aqueous solution which contains an aqueous vehicle such as water as a main component may be used. In addition to water, polyhydric alcohol and water-soluble functional components such as a water-soluble antioxidant or plant extract may be further added within a range in which the effect of the invention is not impaired.

Hereinafter, each components contained in the aqueous cosmetic preparation of the invention will be more specifically described.

1-1. Ceramide analog-containing particle

The ceramide analog-containing particle in the invention contains at least ceramide analog and has a volume average particle diameter of 2 nm to 150 nm which is dispersed in an aqueous phase as an oil-phase component.

The ceramide analog in the invention includes ceramide and derivatives thereof, which may be derived from a synthetic compound or an extracted product.

The term "ceramide analog" as used herein includes a natural ceramide as described below, a compound having a natural ceramide as a basic skeleton, and a precursor which may be derived from these compounds, and is a collective name for a natural ceramide, a glycosylated ceramide such as a sphingoglycolipid, a synthetic ceramide, a sphingosine, a phytosphingosine, and derivatives thereof. Hereinafter, the ceramide analog in the invention will be described in detail.

(Natural ceramides)

The term "natural ceramide" as used herein means a ceramide which has the same structure as that present in the stratum corneum of human skin. Further, a more preferred embodiment of the natural ceramide is that sphingoglycolipid is not contained and three or more hydroxyl groups are included in the molecular structure.

In the following, the natural ceramide used in the invention will be described in detail.

Examples of a fundamental structural formula of the natural ceramide which may be preferably used in the invention are shown in (1-1) to (1-10). (1-1) is known as ceramide 1, (1-2) is known as ceramide 9, (1-3) is known as ceramide 4, (1-4) is known as ceramide 2, (1-5) is known as ceramide 3, (1-6) is known as ceramide 5, (1-7) is known as ceramide 6, (1-8) is known as ceramide 7, (1-9) is known as ceramide 8, and (1-10) is known as ceramide 3B.

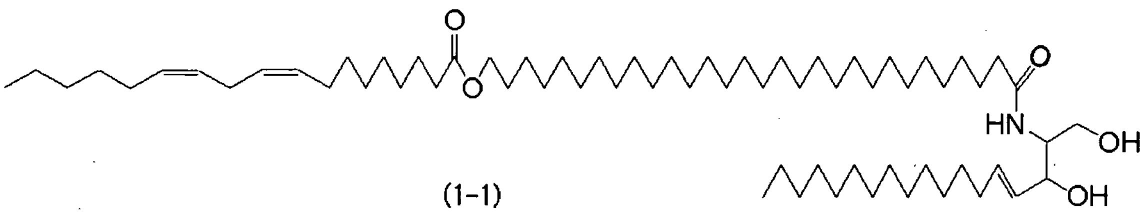

(1-1)

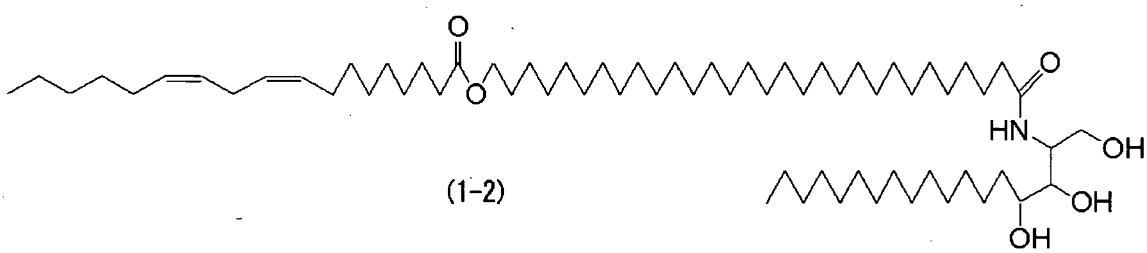

(1-2)

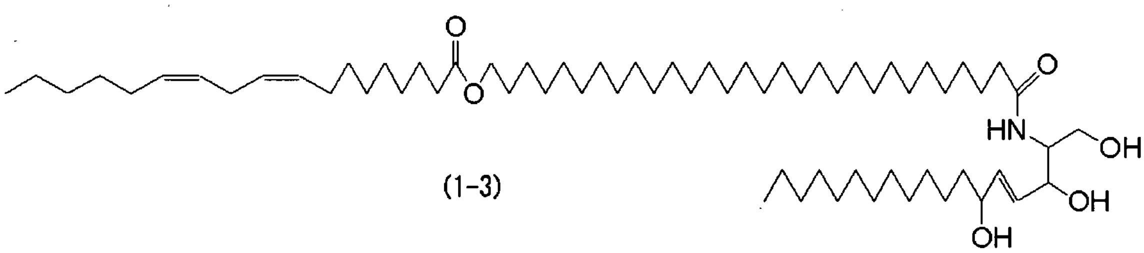

(1-3)

(1-4)

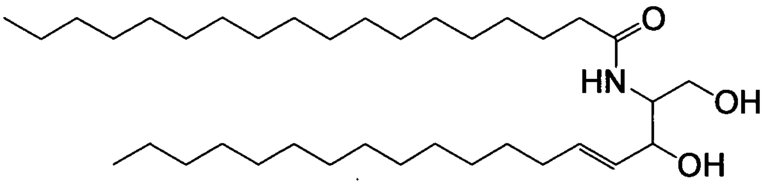

(1-5)

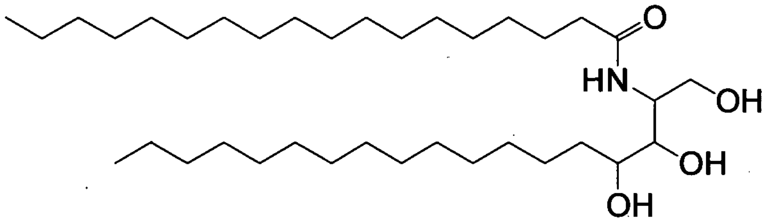

(1-6)

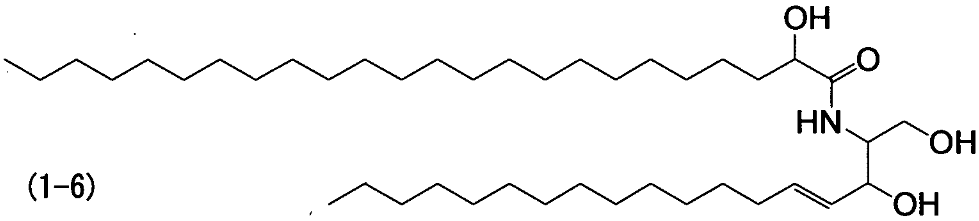

(1-7)

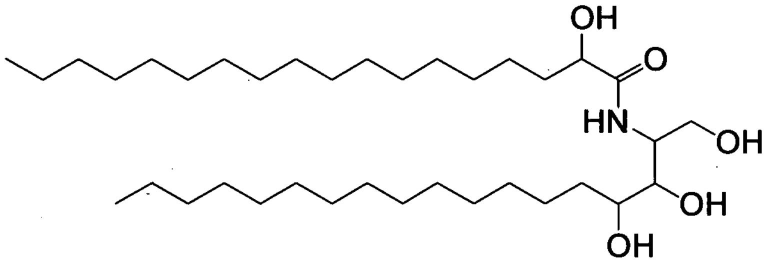

(1-8)

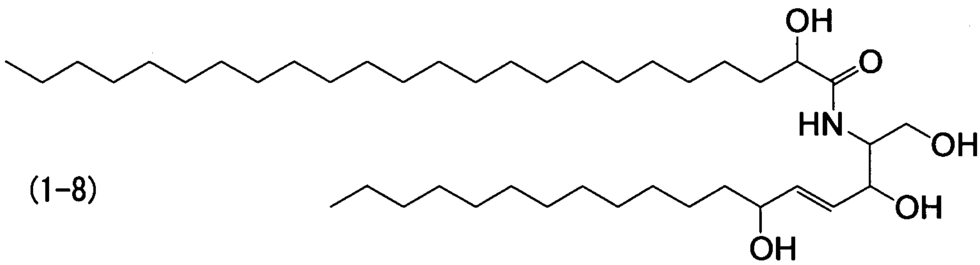

(1-9)

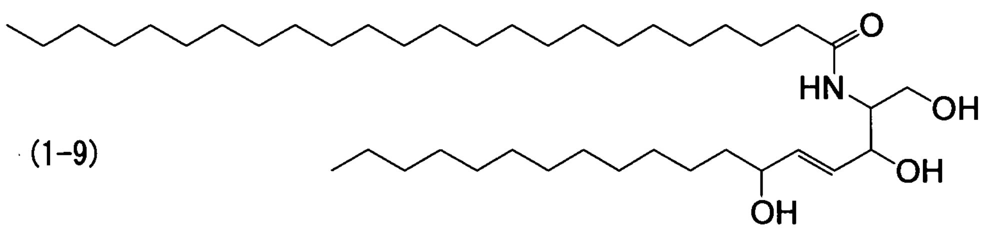

(1-10)

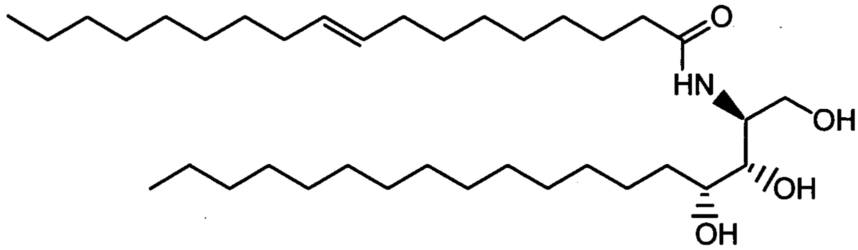

The above structural formula shows one example of each ceramides. Since ceramide is a natural substance, in ceramides actually derived from a human or an animal, there are various variation examples in the length of the alkyl chain, and ceramide having the above skeleton may have any structure in the alkyl chain length.

Alternatively, ceramides modified depending on the purpose such as ceramide in which a double bond is introduced in the molecule in order to impart solubility, and ceramide in which a hydrophobic group is introduced in order to impart permeability, may be used.

Examples of the ceramide having a general structure, which referred to as the natural ceramide, include natural product(extract) and products obtained by microbial fermentation method. Further, synthetic substances and animal-derived substances may also be included.

A natural (D(-) body) optically active body is used as such ceramides. Furthermore, a non-natural (L(+) body) optically active body or a mixture of natural and unnatural type may be used, if necessary. A relative configuration of the above compounds may be natural configuration, or other non-natural configuration, or a mixture thereof.

When the aqueous cosmetic preparation of the invention is used for the purpose of an emollient of a skin, from a viewpoint of the barrier effect, it is preferable to use the natural optically active body.

Such the natural ceramides are also available as a sold product, and examples include Ceramide I, Ceramide III, Ceramide IIIA ceramide IIIB, Ceramide IIIC, and Ceramide VI (all manufactured by Cosmofarm), Ceramide TIC-001 (manufactured by Takasago International Corporation), CERAMIDE II (manufactured by Quest International), DS-Ceramide VI, DS-CLA-Phytoceramide, C6-Phytoceramide, and DS-ceramide Y3S (manufactured by DOOSAN), and CERAMIDE2 (manufactured by Sedama), and the exemplified compound (1-5) is available as trade name: CERAMIDE 3, manufactured by Evonik (formerly Deggusa), and the exemplified compound (1-7) is available as trade name: CERAMIDE 6, manufactured by Evonik (formerly Deggusa).

The natural ceramide which is contained in the ceramide analog-containing particle may be used alone or in combination of two or more thereof. Generally, ceramide analogs have a high melting point and a high crystallinity. Therefore, the combination of two or more natural ceramides is preferable from the viewpoint of emulsion stability and handling performance.

(Glycosylated ceramide)

The glycosylated ceramide is a ceramide compound containing saccharides in the molecule. Examples of the saccharides contained in the molecule of the ceramide compound include monosaccharides such as glucose or galactose; disaccharides such as lactose or maltose; and oligosaccharides and polysaccharides obtained by polymerizing these monosaccharides or disaccharides with a glycoside bond. Further, saccharides may be sugar derivatives in which a hydroxyl group in a sugar unit is replaced with other group. Examples of the sugar derivative include glucosamine, glucuronic acid, and N-acetyl glucosamine. Among them, saccharides having 1 to 5 sugar units are preferable from a viewpoint of dispersion stability. Specifically, glucose and lactose are preferable, and glucose is more preferable.

Specific examples of the glycosylated ceramide include the following compounds.

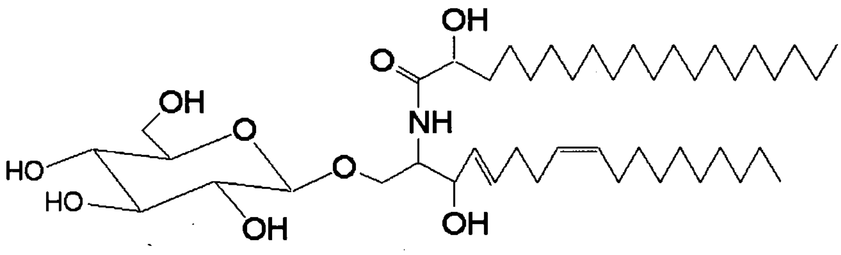

(4-1)

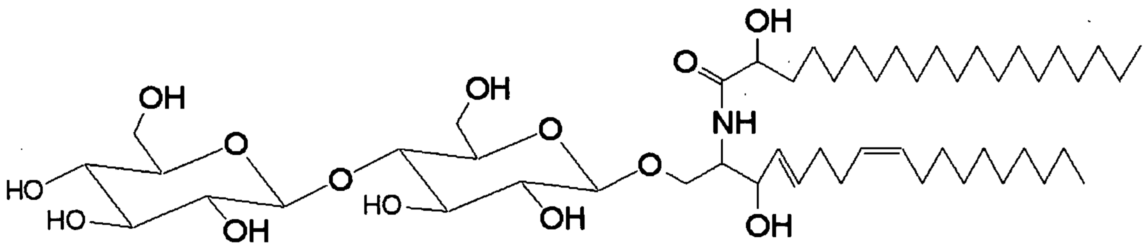

(4-2)

The glycosylated ceramide is available by synthesis or as a commercial product. For example, the exemplified compound (4-1) is available as a trade name: KOME SHINGOGLYCOLIPID manufactured by Okayasu Shoten Co., Ltd..

(Synthetic ceramide)

The synthetic ceramide is synthesized in imitation of the structure of ceramide. As a known compound of such a synthetic ceramide, for example, the synthetic ceramide shown by the following structural formula may be used.

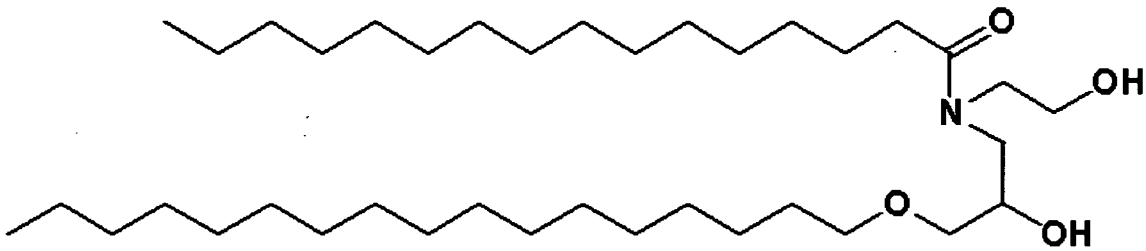

When the synthetic ceramide is used, for example, from a viewpoint of an after-use feeling and a moisturizing feeling upon use of the composition for use of the aqueous cosmetic preparation of the invention, a compound that is synthesized in imitation of the structure of the natural ceramide or the glycosylated ceramide are preferable, and a compound that is synthesized in imitation of the structure of the natural ceramide is more preferable.

(Sphingosine, phytosphingosine)

As sphingosine and phytosphingosine, whether a synthetic product or a natural product, natural sphingosine and a sphingosine analog may be used.

Specific examples of the natural sphingosine include sphingosine, dihydrosphingosine, phytosphingosine, sphingadienine, dehydrosphingosine, dehydrophytosphingosine, and an N-alkylated body (e.g. N-methylated body) thereof, and an acetylated body thereof.

As these sphingosines, a natural (D(-) body) optically active body may be used, or a non-natural (L(+) body) optically active body may be used, or further, a mixture of a natural type and a non-natural type may be used. Relative configuration of the above compound may be natural configuration, may be other non-natural configuration, or may be configuration of a mixture thereof. Among them, examples of phytosphingosine which may be preferably used in the invention include PHYTOSPHINGOSINE

(INCI name; 8th Edition) and exemplified compounds described below.

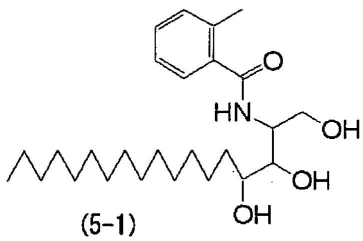

(5-1)

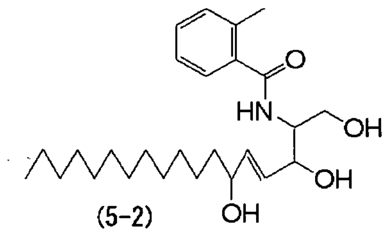

(5-2)

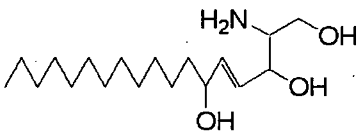

(5-3)

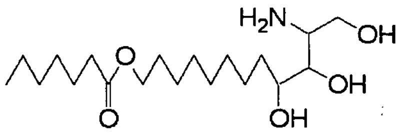

(5-4)

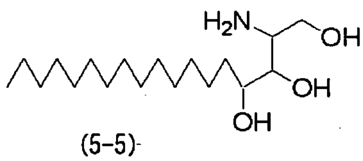

(5-5)

Phytosphingosine may be either a natural extract or a synthetic compound. It may be produced by synthesis or may be available as a commercial product.
Commercially available examples of the natural sphingosine include D-Sphingosine (4-Sphingenine) (manufactured by SIGMA-ALDRICH), D-Sphytosphingosine (manufactured by DOOSAN), phytosphingosine (manufactured by Cosmofarm). Further, Compound (5-5) as exemplified above is available as a trade name of "PHYTOSPHINGOSINE" (manufactured by Evonik (formerly Deggusa)).

Acid

When sphingosines such as sphingosine or phytosphingosine are used in the invention, they are preferably used in combination with a compound having an acidic residue capable of forming a salt with the sphingosines. Preferable examples of the compound having acidic residue include inorganic acids or organic acids having 5 or less carbon atoms.
Examples of the inorganic acid include phosphoric acid, hydrochloric acid, nitric acid, sulfuric acid, perchloric acid, and carbonic acid, and phosphoric acid and hydrochloric acid are preferable.
Examples of the organic acid include monocarboxylic acids such as formic acid, acetic acid, propionic acid, butyric acid, isobutyric acid, and valeric acid; dicarboxylic acids such as succinic acid, phthalic acid, fumaric acid, oxalic acid, malonic acid, and glutaric acid; oxycarboxylic acids such as glycolic acid, citric acid, lactic acid, pyruvic acid, malic acid, and tartaric acid; amino acids such as glutamic acid, and aspartic acid. As these compounds, phosphoric acid,

hydrochloric acid, succinic acid, citric acid, lactic acid, glutamic acid, and aspartic acid are preferable, and lactic acid, glutamic acid, and aspartic acid are particularly preferable.

The acid to be used together may be used by pre-mixing with sphingosines, may be added at the time of formation of the ceramide analogue containing particle, or may be added as a pH adjusting agent after the formation of the ceramide analogue containing particle.

When the acid is used together, the additive amount is preferably about 1 part by mass to 50 parts by mass relative to 100 parts by mass of sphingosines to be used.

(Content of ceramide analog)

From a viewpoint of expecting efficient percutaneous absorption of the ceramide component at the time of using the aqueous cosmetic preparation of the invention as well as the effect originated from ceramide, the content of the ceramide analog is more preferably from 20% by mass to 100% by mass, further preferably form 30% by mass to 100% by mass relative to the total mass of the oil component included in the oil phase in the ceramide analog-containing particle. Thus, it is preferable that the natural ceramide is 30% by mass or more relative to the total mass of the ceramide analog from the viewpoint of expecting the effect of the natural ceramide. It is particularly preferable that the content of the natural ceramide is 100% by mass.

The content of the ceramide analog in the aqueous cosmetic preparation of the invention is preferably in the range of from 0.001 to 5% by mass, and more preferably in the range of from 0.01 to 3% by mass. When the ceramide analog is contained in the aqueous cosmetic preparation within the above-described ranges, moistness and barrier function recovering effects of the aqueous cosmetic preparation of the invention are obtained.

(Particle diameter of ceramide analog-containing particle)

The volume average particle diameter of the ceramide analog-containing particle is from 2 nm to 150 nm, preferably from 3 nm to 150 nm, more preferably from 5 nm to 120 nm, and particularly preferably from 5 nm to 100 nm. When the particle diameter of the ceramide analog-containing particle is from 3 nm to 150 nm, the transparency of the aqueous cosmetic preparation is ensured and the effects that are desired for aqueous cosmetic preparations, for example skin penetration, may be well exerted.

The particle diameter of the ceramide analog-containing particle may be measured with a commercially available particle size distribution meter.

Known examples of the method for measuring particle size distribution include optical microscopy, a confocal laser scanning microscope method, an electron microscopic method, atomic force microscopy, a static light scattering method, laser diffractometry, dynamic light scattering, a centrifugal sedimentation method, electric pulse measurement, a chromatography method, and an ultrasonic attenuation method. Apparatuses based on each of these principles are commercially available.

In the measurement of the particle diameter of the ceramide analog-containing particle in the invention, it is preferable to use the dynamic light scattering from the viewpoint of particle diameter range and ease of measurement. Examples of commercially available measuring apparatus using dynamic light scattering include a Nanotrac UPA (manufactured by Nikkiso Co., Ltd.), a dynamic light scattering particle size distribution meter LB-550 (manufactured by HORIBA Ltd.), and a concentrated-system particle diameter analyzer FPAR-1000 (manufactured by Otsuka Electronics Co., Ltd.).

The particle diameter of ceramide analog-containing particle in the invention is a value measured by using the dynamic light scattering particle size distribution meter LB-550 (manufactured by HORIBA Ltd.). Specifically, a value measured in the following manner is employed.

That is, in the method for measuring the particle diameter of the ceramide analog-containing particle, a sample divided from the aqueous cosmetic preparation of the invention is diluted with pure water so that the concentration of the oil component contained in the sample is 1% by mass and the particle diameter is measured using a quartz cell. The particle diameter may be determined as a median diameter when the refractive index of a sample is 1.600, the refractive index of a dispersion medium is 1.333 (pure water), and the viscosity of pure water is set as the viscosity of the dispersion medium.

Embodiments of the process of forming the ceramide analog-containing particle include:

1) a process of forming ceramide analog-containing particles (oil phase) in advance as solid particles and then dispersing these in a dispersion medium (aqueous phase); and
2) a process of forming a ceramide analog-containing particle in the system by heating a ceramide analog to change it into a molten state or dissolving a ceramide analog in an appropriate solvent to change it into a liquid state and then adding the resultant to the aqueous phase to disperse it, followed by reducing the temperature to ordinary

ambient temperature or removing the solvent. Further, it is preferable that the natural ceramide or the like is prepared so as to be soluble in another oil component or is prepared by dissolving it in an organic solvent.

(Other oil components)

The aqueous cosmetic preparation of the invention is formed by dispersing a ceramide analog-containing particle in an aqueous phase as an oil phase. The aqueous cosmetic preparation may also have a configuration in which an oil component and/or solvent (may be referred to as "another(other) oil component(s)" in the present specification) different from the ceramide analog such as natural ceramide as described above is contained in the oil phase, and an oil droplet-like dispersed particle containing natural ceramide is present as a natural ceramide-containing particle in the oil component and/or solvent. When this embodiment is used, the average particle diameter of the ceramide analog-containing particle in the invention means the average particle diameter of an oil droplet-like dispersed particle which contains a ceramide analog-containing particle.
In this regard, the term "another oil component" refers to the oil component which is not separated from the ceramide analog at an ordinary temperature. The term "solvent" refers to the solvent which may dissolve the ceramide analog, and examples thereof include alcohols.
Here, other oil components to be used together in the invention are not particularly limited. Other oil components may be, for example, oil components that are active components which are added in accordance with the intended use of the aqueous cosmetic preparation. Further, they may be oil components which are used to improve the dispersion stability and after-use feeling of the skin and control physical properties of the aqueous cosmetic preparation. Hereinafter, other oil components to be used in the invention will be described. In this regard, a fatty acid having 10 to 30 carbon atoms of the "(2) fatty acid having 10 to 30 carbon atoms or salt thereof" in the invention may be included in the ceramide analog-containing particle as another oil component. The fatty acid having 10 to 30 carbon atoms or salt thereof will be described later, in detail.

(Oil component as an active ingredient)

In the invention, it is preferable that a functional ingredient for cosmetics which is insoluble or hardly-soluble in an aqueous vehicle, particularly water, is included as an oil component. When the aqueous cosmetic preparation of the invention contains functional oil ingredients such as carotenoids, which are described later, an excellent emollient effect, an anti-aging effect of the skin, and an anti-oxidant effect are given to the aqueous cosmetic preparation of the invention.
The term "functional ingredient" used herein means an ingredient which may be expected to be induce a certain physiological effect in a living body when the ingredient is applied to or introduced into the living body.
The oil component which may be used in the invention is not particularly limited as far as it is a component which is insoluble or hardly soluble in an aqueous medium, particularly water, but a radical scavenger containing an oil-soluble vitamin such as carotenoids and tocopherols, or fats or oils such as coconut oil are preferably used.
The term "insoluble in an aqueous vehicle" means that the solubility in 100 mL of an aqueous vehicle is 0.01 g or less at 25°C. The term "hardly-soluble in an aqueous vehicle means that the solubility in 100 mL of an aqueous vehicle is more than 0.01 g and 0.1 -g or less at 25°C.

Carotenoids

As the oil component, carotenoids including a natural colorant may be preferably used.
Carotenoids which may be used in the aqueous cosmetic preparation of the invention are colorants of terpenoids ranging in color from yellow to red, and include natural substances such as plants, algae, and bacteria.
Further, carotenoids are not limited to naturally-derived substances. Any carotenoids are included in carotenoids in the invention as long as they are obtained according to the conventional method. For example, many of carotenes of carotenoids described later are also produced by synthesis, and many of commercially available β-carotenes are produced by synthesis.
Examples of the carotenoids include hydrocarbons (carotenes) and oxidized alcohol derivatives thereof (xanthophylls).
Examples carotenoids include actinioerythrol, bixin, canthaxanthin, capsanthin, capsorbin, β-8'-apo-carotenal (apocarotenal), β-12'-apo'-carotenal, α-carotene, β-carotene, "carotene" (mixtures of α- and β-carotenes), γ-carotene, β-cryptoxanthin, lutem, lycopene, violaxanthin, zeaxanthin, and esters of them which have a hydroxyl or carboxyl group.
Many of carotenoids exist in nature in the form of cis- and trans-isomers, and synthetic products are often cis-trans- mixtures.
Carotenoids may be generally extracted from plant materials. These carotenoids have various functions and,

for example, lutein extracted from a petal of marigold is widely used as a raw material of a feed of poutly, and has the function of coloring a skin of poutly, and lipid, as well as an egg laid by poutly.

The carotinoides may be contained in the ceramide analog-containing particle. In addition, the carotinoides may be contained in the aqueous cosmetic preparation separately from the ceramide analog-containing particle.

Fats or oils

Examples of fats or oils used as other oil component include fats or oils which are liquid at a normal temperature (fatty oils) and fats or oils which are solid at a normal temperature (fats).

Examples of liquid fats or oils include an olive oil, a camellia oil, a macadamia nut oil, a castor oil, an avocado oil, an evening primrose oil, a turtle oil, a corn oil, a mink oil, a rapeseed oil, an egg yolk oil, a sesame oil, a persic oil, a wheat germ oil, a sasanqua oil, a flaxseed oil, a cotton seed oil, a perilla oil, a soybean oil, an arachis oil, a tea seed oil, a kaya oil, a rice bran oil, a chinese wood oil, a Japanese tung oil, jojoba oil, an embryo oil, a triglycerol, a glyceryl trioctanoate, a glycerin triisopalmitate, a salad oil, a safflower oil (Carthamus tinctorius oil), a palm oil, a coconut oil, a peanut oil, an almond oil, a hazelnut oil, a walnut oil, and a grape seed oil.

Examples of the solid fats or oils include a beef tallow, a hardened beef tallow, a neatsfoot oil, a beef bone fat, a mink oil, an egg yolk oil, a lard, a horse fat, a mutton tallow, a hardened oil, a cacao butter, a palm oil, a hardened palm oil, a palm oil, a palm hardened oil, a Japan wax, a Japan wax kernel oil, and a hardened castor oil.

Among them, a coconut oil which is a medium chain fatty acid triglyceride is preferably used from a viewpoint of the dispersed particle diameter and stability of the aqueous cosmetic preparation.

In the invention, as the fats or oils, commercially available products may be used. Further, in the invention, the fats or oils may be used alone, or may be used by mixing them.

Examples of a compound having a phenolic hydroxyl group which may be used as other oil component in the invention include polyphenols (e.g. catechin), guaiac butter, nordihydroguaretic acid (NDGA), gallic acid esters, BHT (butylhydroxytomene), BHA (butylhydroxyanisole), vitamin Es and bisphenols. Examples of gallic acid esters include propyl gallate, butyl gallate and octyl gallate.

Examples of the amine compound include phenylenediamine, diphenyl-p-phenylenediamine and 4-amino-p-diphenylamine, and diphenyl-p-phenylenediamine or 4-amino-p-diphenylamine is more preferable.

Examples of an oil-solubilized derivative of ascorbic acid or erythorbic acid include L-ascorbyl stearate, L-ascorbyl tetraisopalmitate, L-ascorbyl palmitate, erisorbyl palmitate, and erisorbyl tetraisopalmitate.

Among them, a vitamin E group is particularly preferably used from a viewpoint of excellence in safety and function of antioxidant.

The vitamin E group is not particularly limited. Examples of the vitamin E group include a compound group consisting of tocopherol and derivatives thereof, as well as a compound group consisting of tocotrienol and derivatives thereof. These may be used alone or in combination with a plurality of them. Alternatively, the compound selected from the group consisting of tocopherol and derivatives thereof may be used in combination with the compound selected from the group consisting of tocotrienol and derivatives thereof.

Examples of the compound group consisting of tocopherol and derivatives thereof include dl-α-tocopherol, dl-β-tocopherol, dl-γ-tocopherol, dl-α-tocopherol, acetic acid dl-α-tocopherol, nicotinic acid-dl-α-tocopherol, linolic acid-dl-α-tocopherol, and succinic acid dl-α-tocopherol. Among them, dl-α-tocopherol, dl-β-tocopherol, dl-γ-tocopherol, dl-α-tocopherol, and mixtures thereof (mixed tocopherol) are more preferable. As tocopherol derivatives, acetic esters of them are preferably used.

Examples of the compound group consisting of tocotrienol and derivatives thereof include α-tocotrienol, β-tocotrienol, γ-tocotrienol, and σ-tocotrienol. As tocotrienol derivatives, acetic esters thereof are preferably used. Tocotrienol is a tocopherol analog included in wheat, rice bran, and palm oil, has three double bonds in the side chain of tocopherol, and shows excellent antioxidant performance.

The vitamin E group are particularly contained, in the oil phase of the aqueous cosmetic preparation as the oil-soluble antioxidant since the antioxidant function of the oil component may be effectively exhibited. Among the vitamin E group, at least one compound selected from the compound group consisting of tocotrienol and derivatives thereof is preferably contained from a viewpoint of the oxidation preventing effect.

The content of other oil component such as carotinoids and fats or oils may be suitably determined according to the formulation of the aqueous cosmetic preparation of the invention.

1-2. Fatty acid having 10 to 30 carbon atoms or salt thereof

The aqueous cosmetic preparation of the invention contains fatty acid having 10 to 30 carbon atoms or a salt thereof (hereinafter, may be referred to as the "fatty acid component"). When the aqueous cosmetic preparation is prepared, such fatty acid components are easily dissolved into the system in the process of mixing an oil phase component

and an aqueous phase component, thereby excellent dispersion stability of a fine ceramide analog-containing particle included in the aqueous cosmetic preparation is exhibited. Thus, the transparency is not impaired, for example, in a case where transparency is required for the aqueous cosmetic preparation.

When the fatty acid having 10 to 30 carbon atoms is used as the fatty acid component, the fatty acid is contained in the aqueous cosmetic preparation as an oil phase component, and is preferably contained as one of a structural component of the ceramide analog-containing particle.

Further, when the salt of fatty acid (fatty acid salt) having 10 to 30 carbon atoms is used as the fatty acid component, the fatty acid salt may be an aqueous phase component of the aqueous cosmetic preparation since the fatty acid salt is soluble in the aqueous vehicle. As the fatty acid component in the invention, the fatty acid having 10 to 30 carbon atoms or salt thereof may be used alone or in combination thereof.

The fatty acid having 10 to 30 carbon atoms may be either a saturated or unsaturated fatty acid. From a viewpoint of emulsification and dispersion stability, it is preferable that the fatty acid having 10 to 30 carbon atoms is in liquid form at 30°C.

Specific examples of the fatty acid component in the invention include capric acid, lauric acid, myristic acid, palmitic acid, stearic acid, oleic acid, 12-hydroxy stearic acid, undecylenic acid, tolic acid, isostearic acid, arachidic acid, behenic acid, linolic acid, α-linolenic acid, γ-linolenic acid, arachidonic acid, docosahexaenoic acid (DHA), eicosapentaenoic acid (EPA), erucic acid, and respective salts thereof. These components may be used alone or in combination of two or more thereof. From a viewpoint of color, smell, and skin irritation, the fatty acid component in the invention is preferably at least one kind selected from the group consisting of lauric acid, isostearic acid, oleic acid, γ-linolenic acid, α-linolenic acid, and respective salts thereof, particularly preferably oleic acid.

When the fatty acid salt is used as the fatty acid component, examples of the salt structure constituting the fatty acid salt include metal salts such as sodium or potassium; basic amino acid salts such as L-arginine, L-histidine, or L-lysine; and alkanolamine salts such as triethanolamine. The type of salt is suitably selected in accordance with the type of fatty acid to be used. From a viewpoint of solubility and dispersibility, metal salt such as sodium salt is preferable.

The amount of the fatty acid component contained in the aqueous cosmetic preparation of the invention is preferably the amount which may disperse the ceramide analog sufficiently. From a viewpoint of preservation stability and transparency, the amount is preferably from 0.01 to 1.0 times of the total mass of the ceramide analog. From a viewpoint of preservation stability, the amount is preferably from 0.05 to 0.5 times of the total mass of the ceramide analog. When the amount of the fatty acid component is 1.0 times or less of the total mass of the ceramide analog, it is preferable in that excessive separation or precipitation of fatty acid is suppressed. On the other hand, when the amount of the fatty acid component is 0.01 times or more of the total mass of the ceramide analog, it is preferable in that the fixation of the fatty acid component to the ceramide analog is sufficient.

From a viewpoint of the transparency of the aqueous cosmetic preparation, the content of the fatty acid component is preferably from 0.00001% by mass to 3.0% by mass, more preferably from 0.00005% by mass to 2.0% by mass relative to the total mass of the aqueous cosmetic preparation.

1-3. Surfactant

The aqueous cosmetic preparation of the invention may contain a surfactant. The above-described fatty acid component is not included in the surfactant.

Examples of the surfactant other than the fatty acid component in the invention include cationic, anionic, amphoteric, and nonionic surfactants.

Examples of the nonionic surfactant include glycerine fatty acid ester, organic acid monoglyceride, polyglycerin fatty acid ester, propylene glycol fatty acid ester, polyglycerin condensed ricinoleic acid ester, sorbitan fatty acid ester, sucrose fatty acid ester, and polyoxyethylene sorbitan fatty acid ester. These nonionic surfactants may be contained as an oil-phase component in the aqueous cosmetic preparation of the invention.

Among the nonionic surfactants, polyglycerin fatty acid ester is preferable from a viewpoint of emulsion stability. Particularly, polyglycerin fatty acid ester with an HLB of form 10 to 16 (hereinafter, may be referred to as the "specific polyglycerin fatty acid ester") is more preferable. The polyglycerin fatty acid ester may be contained in the oil phase.

Surfactants such as the specific polyglycerin fatty acid ester is preferable since the specific polyglycerin fatty acid ester may reduce the interfacial tension of oil phase/aqueous phase greatly, and thereby the particle diameter of the ceramide analog-containing particle which is contained in the aqueous cosmetic preparation as an oil phase may be smaller.

Herein, HLB is hydrophilicity-hydrophobicity balance which is usually used in the field of surfactants, and a calculation equation which is usually used, for example, Kawakami equation may be used. In the invention, the following Kawakami equation is adopted.

$$HLB = 7 + 11.7 \log (M_w/M_o)$$

In the equation, $M_w$ is the molecular weight of a hydrophilic group, and $M_o$ is the molecular weight of a hydrophobic group.

Alternatively, numerical values of HLB described in catalogs may be used. As is apparent from the aforementioned equation, a surfactant of an arbitrary HLB value may be obtained by utilizing additivity of HLB.

As for the preferable examples of the polyglycerin fatty acid ester, it is particularly preferable that at least one of them is an ester of a polyglycerin with an average degree of polymerization of 10, and a fatty acid having 8 to 18 carbon atoms (for example, caprylic acid, capric acid, lauric acid, myristic acid, Barh Myzin acid, stearic acid, oleic acid, and linolic acid).

Preferable examples of the polyglycerin fatty acid ester include hexaglycerol monooleate, hexaglycerol monopalmitate, hexaglycerol monomyristate, hexaglycerol monolaurate, decaglycerol monooleate, decaglycerol monostearate, decaglycerol monopalmitate, decaglycerol monomyristate, and decaglycerol monolaurate. The HLB values of these compounds are from 10 to 16. Among them, decaglycerol monolinoleate (HLB = 12), decaglycerol monooleate (HLB = 12), decaglycerol monostearate (HLB = 12), decaglycerol monopalmitate (HLB = 13), decaglycerol monomyristate (HLB = 14), and decaglycerol monolaurate (HLB = 16) are more preferable.

As the polyglycerin fatty acid ester, decaglycerol oleate is particularly preferable. In the invention, the specific polyglycerin fatty acid ester may be used alone or in combination of two or more thereof.

As for the surfactant in the invention, one selected from polyglycerin fatty acid ester with an HLB of from 10 to 16 and one or more selected from polyglycerin fatty acid ester with an HLB of from 5 to 15 which has a molecular structure different from the former polyglycerin fatty acid ester may be combined. In this regard, the polyglycerin fatty acid ester with an HLB of from 5 to 15 may be polyglycerin fatty acid ester which is contained in polyglycerin fatty acid esters as described above or may be other polyglycerin fatty acid esters.

In the invention, a preferred embodiment contains, as the surfactant, decaglycerol oleate and polyglycerin fatty acid ester in which the polymerization degree of glycerol is less than 10 and the number of carbon atom in fatty acid is from 12 to 18. A more preferable example of polyglyceryl fatty acid ester in which the degree of polymerization of the glycerol is less than 10 and the number of carbon atoms in fatty acid is from 12 to 18 includes polyglyceryl fatty acid ester which is at least one selected from hexaglycerin fatty acid ester and tetraglycerin fatty acid ester and has an HLB value of form 5.0 to 15.

Examples of hexaglycerin fatty acid ester and tetraglycerin fatty acid ester which are suitably used together with decaglycerol oleate include tetraglycerol monostearate (HLB = 6), tetraglycerol monooleate (HLB = 6), hexaglycerol monolaurate (HLB = 14.5), hexaglycerol monomyristate (HLB = 11), hexaglycerol monostearate (HLB = 9), and hexaglycerol monooleate (HLB = 9).

When decaglycerol oleate is used in combination with hexaglycerin fatty acid ester and/or tetraglycerin fatty acid ester in the invention, the content ratio may be properly determined according to the application form of the ceramide dispersion and (decaglycerin fatty acid ester) / (tetraglycerin fatty acid ester and/or hexaglycerin fatty acid ester) is preferably from 1/0 to 1/1, more preferably 1/0.5, and further preferably 1/0.25.

A commercially available product of polyglycerin fatty acid ester such as a specific polyglycerin fatty acid ester may be used.

Examples of the commercially available product of polyglycerin fatty acid ester include NIKKOL DGMS, NIKKOL DGMO-CV, NIKKOL DGMO-90V, NIKKOL DGDO, NIKKOL DGMIS, NIKKOL DGTIS, NIKKOL Tetraglyn 1-SV, NIKKOL Tetraglyn 1-O, NIKKOL Tetraglyn 3-S, NIKKOL Tetraglyn 5-S, NIKKOL Tetraglyn 5-O, NIKKOL Hexaglyn 1-L, NIKKOL Hexaglyn 1-M, NIKKOL Hexaglyn 1-SV, NIKKOL Hexaglyn 1-O, NIKKOL Hexaglyn 3-S, NIKKOL Hexaglyn 4-B, NIKKOL Hexaglyn 5-S, NIKKOL Hexaglyn 5-0, NIKKOL Hexaglyn PR-15, NIKKOL Decaglyn 1-L, NIKKOL Decaglyn 1-M, NIKKOL Decaglyn 1-SV, NIKKOL Decaglyn 1-50SV, NIKKOL Decaglyn 1-ISV, NIKKOL Decaglyn 1-O, and NIKKOL Decaglyn 1-OV, NIKKOL Decaglyn 1-LN, NIKKOL Decaglyn 2-SV, NIKKOL Decaglyn 2-ISV, NIKKOL Decaglyn 3-SV, NIKKOL Decaglyn 3-OV, NIKKOL Decaglyn 5-SV, NIKKOL Decaglyn 5-HS, NIKKOL Decaglyn 5-IS, NIKKOL Decaglyn 5-OV, NIKKOL Decaglyn 5-O-R, NIKKOL Decaglyn 7-S, NIKKOL Decaglyn 7-O and NIKKOL Decaglyn 10-SV, NIKKOL Decaglyn 10-IS, NIKKOL Decaglyn 10-OV, NIKKOL Decaglyn 10-MAC, and NIKKOL Decaglyn PR-20 (manufactured by Nikko Chemicals Co., Ltd.)

Ryoto Polygly Ester, L-7D, L-10D, M-10D, P-8D, SWA-10D, SWA-15D, SWA-20D, S-24D, S-28D, O-15D, O-50D, B-70D, B-100D, ER-60D, LOP-120DP, DS13W, DS3, HS11, HS9, TS4, TS2, DL15, and DO13 (manufactured by Mitsubishi-Kagaku Foods Corporation); Sunsoft Q-17UL, Sunsoft Q-14S, and Sunsoft A-141C (manufactured by Taiyo Kagaku Co., Ltd.); and Poem DO-100 and Poem J-0021 (manufactured by Riken Vitamin Co., Ltd.).

Among them, NIKKOL Decaglyn 1-L, NIKKOL Decaglyn 1-M, NIKKOL Decaglyn 1-SV, NIKKOL Decaglyn 1-50SV, NIKKOL Decaglyn 1-ISV, NIKKOL Decaglyn 1-O, and NIKKOL Decaglyn 1-OV, NIKKOL Decaglyn 1-LN, Ryoto Polygly Ester, L-7D, L-10D, M-10D, P-8D, SWA-10D, SWA-15D, SWA-20D, S-24D, S-28D, O-15D, O-50D, B-70D, B-100D, ER-60D, and LOP-120DP are preferable.

Other examples of the nonionic surfactant includes other glycerin fatty acid esters, organic acid monoglyceride, polyglycerin fatty acid ester, propylene glycol fatty acid ester, polyglycerin condensed ricinoleic acid ester, sorbitan fatty acid ester, sucrose fatty acid ester, and polyoxyethylene sorbitan fatty acid ester. Sorbitan fatty acid ester, sucrose fatty acid ester, and polyoxyethylene sorbitan fatty acid ester are more preferable. Further, these surfactants are not necessarily required to be highly purified by distillation and they may be reaction mixtures.

As for sorbitan fatty acid ester, the number of carbon atoms in fatty acid is preferably 8 or more, more preferably 12 or more. Preferable examples of sorbitan fatty acid ester include sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate, and sorbitan trioleate. In the invention, these sorbitan fatty acid esters may be used alone, or may be used by mixing them.

Examples of the commercially available product of sorbitan fatty acid ester include NIKKOL SL-10 and SP-10V, SS-10V, SS-10MV, SS-15V, SS-30V, SI-10RV, SI-15RV, SO-10V, SO-15MV, SO-15V, SO-30V, SO-10R, SO-15R, SO-30R, and SO-15EX (manufactured by Nikko Chemicals Co., Ltd.); Solgen 30V, 40V, 50V, 90, and 110 (manufactured by DAI-ICHI KOGYO SEIYAKU CO., LTD.); and RHEODOL AS-10V, AO-10V, AO-15V, SP-L10, SP-P10, SP-S10V, SP-S30V, SP-O10V, and SP-O30V (manufactured by Kao Corporation).

As for sucrose fatty acid ester, the number of carbon atom in fatty acid is preferably 12 or more, more preferably from 12 to 20.

Preferable examples of sucrose fatty acid ester include sucrose dioleate, sucrose distearate, sucrose dipalmitate, sucrose dimyristate, sucrose dilaurate, sucrose monooleate, sucrose monostearate, sucrose monopalmitate, sucrose monomyristate, and sucrose monolaurate. Among them, sucrose monooleate, sucrose monostearate, sucrose monopalmitate, sucrose monomyristate, and sucrose monolaurate are more preferable.

In the invention, these sucrose fatty acid esters may be used alone, or may be used by mixing them.

Examples of the commercial product of sucrose fatty acid ester include Ryoto sugar ester S-070, S-170, S-270, S-370, S-370F, S-570, S-770, S-970, S-1170, S-1170F, S-1570, S-1670, P-070, P-170, P-1570, P-1670, M-1695, 0-170, 0-1570, OWA-1570, L-195, L-595, L-1695, LWA-1570, B-370, B-370F, ER-190, ER-290, and POS-135 (manufactured by Mitsubishi-Kagaku Foods Corporation); and DK Ester SS, F160, F140, F110, F90, F70, F50, F-A50, F-20W, F-10, F-A10E, Cosmelike B-30, S-10, S-50, S-70, S-110, S-160, S-190, SA-10, SA-50, P-10, P-160, M-160, L-10, L-50, L-160, L-150A, L-160A, R-10, R-20, O-10, and O-150 (manufactured by DAI-ICHI KOGYO SEIYAKU CO., LTD.).

Among them, Ryoto sugar ester S-1170, S-1170F, S-1570, S-1670, P-1570, P-1670, M-1695, 0-1570, L-1695, DK Ester SS, F160, F140, F110, Cosmelike S-110, S-160, S-190, P-160, M-160, L-160, L-150A, L-160A, and O-150 are preferable.

As for polyoxyethylene sorbitan fatty acid ester, the number of carbon atoms of fatty acid is preferably 8 or more, more preferably 12 or more. The length (the number of addition mole) of ethyleneoxide of polyoxyethylene is preferably 2 to 100, more preferably 4 to 50.

Preferable examples of polyoxyethylene sorbitan fatty acid ester include polyoxyethylene sorbitan monocaprylate, polyoxyethylene sorbitan monolaurate, polyoxyethylene sorbitan monostearate, polyoxyethylene sorbitan sesquistearate, polyoxyethylene sorbitan tristearate, polyoxyethylene sorbitan isostearate, polyoxyethylene sorbitan sesquiisostearate, polyoxyethylene sorbitan oleate, polyoxyethylene sorbitan sesquioleate, and polyoxyethylene sorbitan trioleate.

The polyoxyethylene sorbitan fatty acid esters may be used alone, or may be used by mixing them.

Examples of the commercially available product of polyoxyethylene sorbitan fatty acid ester include NIKKOL TL-10, NIKKOL TP-10V, NIKKOL TS-10V, NIKKOL TS-10MV, NIKKOL TS-106V, NIKKOL TS-30V, NIKKOL TI-10V, NIKKOL TO-10V, NIKKOL TO-10MV, NIKKOL TO-106V, and NIKKOL TO-30V (manufactured byNikko Chemicals Co., Ltd.); RHEODOL TW-L106, TW-L120, TW-P120, TW-S106V, TW-S120V, TW-S320V, TW-O106V TW-O120V, TW-O320V TW-IS399C, RHEODOL SUPER SP-L10, and TW-L120 (manufactured by Kao Corporation); and SORGEN TW-20, TW-60V, and TW-80V (manufactured by DAI-ICHI KOGYO SEIYAKU CO., LTD.).

1-4. Polyhydric alcohol

It is preferable that the aqueous cosmetic preparation of the invention contains polyhydric alcohol from a viewpoint of the particle diameter of the ceramide analog-containing particle, dispersion stability, preservation stability, and antiseptic properties.

The polyhydric alcohol has the moisturizing function and the viscosity adjusting function. In addition, the polyhydric alcohol also has the function of reducing the interface tension between water and a fat or oil component, making an interface easily spread, and making easier to form a fine and stable particle.

From the foregoing, inclusion of the polyhydric alcohol in the aqueous cosmetic preparation is preferable from a viewpoint that the dispersed particle diameter of the aqueous cosmetic preparation may be finer, and the particle diameter may be stably retained for a long period of time in the state where the particle diameter is fine.

In addition, by addition of the polyhydric alcohol, the moisture activity of the aqueous cosmetic preparation may be reduced, and proliferation of microorganisms may be suppressed.

The polyhydric alcohol which may be used in the invention is not particularly limited, as long as it is a di- or more hydric alcohol.

Examples of polyhydric alcohol include glycerin, diglycerin, triglycerin, polyglycerin, 3-methyl-1,3-butanediol, 1,3-butylene glycol, isoprene glycol, polyethylene glycol, 1,2-pentanediol, 1,2-hexandiol, propylene glycol, dipropylene glycol, polypropylene glycol, ethylene glycol, diethylene glycol, pentaerythritol, neopentyl glycol, maltitol, reduced starch syrup, saccharose, lactitol, palatinit, erythritol, sorbitol, mannitol, xylitol, xylose, glucose, lactose, mannose, maltose, galactose, fructose, inositol, pentaerythritol, maltotriose, sorbitan, trehalose, amylolysis sugar, and amylolysis sugar reduced alcohol. These compounds may be used alone, or in the form of a mixture of plural kinds.

Further, it is preferable to use a polyhydric alcohol having 3 or more hydroxyl groups per molecule. Thereby, the interface tension between an aqueous solvent and a fat or oil component may be more effectively reduced, and a finer and stable particle may be formed. As a result, for example, when the aqueous cosmetic preparation of the invention is applied to the skin, the skin penetration is further increased.

Among polyhydric alcohols satisfying the aforementioned conditions, particularly when glycerin is used, the oil droplet particle diameter of the topical composition for external use becomes more smaller, and the particle is stably retained for a long period of time while the particle diameter is small, being preferable.

From a viewpoint of the viscosity of nano ceramide dispersion and composition in addition to the particle diameter, stability, and antiseptic properties, the content of the polyhydric alcohol is preferably from 1% by mass to 20% by mass, more preferably from 2% by mass to 18% by mass, further preferably from 32% by mass to 15% by mass relative to the total mass of the aqueous cosmetic preparation.

When the content of the polyhydric alcohol is 1% by mass or more, it is preferable in that sufficient preservation stability and moistness are easily obtained depending on the kind and the content of the fat or oil component. On the other hand, when the content of the polyhydric alcohol is more than 20% by mass, stinging (tingling feeling of the skin) is caused. Therefore, preferable content of the polyhydric alcohol is 20% by mass or less.

1-5. Polymeric compound

The aqueous cosmetic preparation of the invention may contain a polymeric compound. Examples of the polymeric compound include a water-soluble polymer compound, an amphiphilic polymer, and a non-water-soluble polymer.

Any kind of synthetic polymers, natural polymers, and semi-synthetic polymers may be used as the water-soluble polymer compound. Particularly, saccharides, proteins, and complexes thereof are preferable.

Examples of saccharides include monosaccharides, disaccharides, oligosaccharides, polysaccharides, dextrin, derivatives of starch, gums, mucopolysaccharides, and celluloses, however, the invention is not limited thereto.

Typical examples thereof include agarose, arabinose, amylose, amylopectin, acacia gum, gum arabic, arabinogalactan, alkyl glycoside, alginic acid, sodium alginate, propylene glycol alginate, aldose, inulin, oligosaccharide, gatti gum, curdlan, carrageenan, galactomannan, galactose, xanthan gum, xylose, xyloglucan, chitin, chitosan, Cyamoposis Gum, cluster dextrin, β-glucan, glucuronic acid, glycogen, glycosaminoglycan, glyceraldehyde, glucosamine, glucose, glucomannan, ketose, chondroitin sulfate, psyllium seed gum, gellant gum, cyclodextrin, sucrose, hydroxyethyl cellulose, hydroxypropylcellulose, carboxymethylcellulose, methyl cellulose, cellobiose, sorbitol, deoxyribose, dextrin, invert sugar, starch, soybean polysaccharides, sugar-alcohol, glycoprotein, tragacanth gum, trehalose, hyaluronic acid, fucose, fructose, pullulan, pectin, heparin, hemicellulose, maltose, mannitol, mannan, lactose, and ribose. However, the invention is not limited thereto.

Among these saccharides, gums and polysaccharides are preferable from a viewpoint of dispersion stability caused by an increase in the viscosity, and xanthan gum, gum arabic, and pullulan are more preferable from a viewpoint of stability of carotenoids.

Furthermore, any kind of proteins may be used as long as it is a polymer or an oligomer in which an amino acid is polymerized with a peptide bond. However, the proteins naturally derived and water-soluble are more preferable.

There are a simple protein consisting of an amino acid, and a composite protein containing a structural component other than the amino acid. Both proteins may be used. Examples of the simple protein include gelatin, casein, fibroin, sericin, keratin, and protamine. Examples of the composite protein include glycoprotein which is a protein bonded to a carbohydrate, lipoprotein which is a protein bonded to a lipid, metalloprotein which is a protein bonded to a metal ion, nucleoprotein which is a protein bonded to a ribonucleic acid, and phosphoprotein which is a protein bonded to a phosphate group.

On the other hand, generally, there are many proteins called from a protein raw material. Examples thereof include an animal muscle protein, a milk protein, an egg protein, a rice protein, a wheat protein (wheat gluten), a soybean protein, a yeast protein, a bacterial protein.

In this regard, such proteins may be used as mixtures.

Further, it is also preferable that polymers (e.g., collagen, hyaluronic acid, and elastin) which are present in the skin may be added in the cosmetic preparation. The polymeric compounds as described above may be used alone or in combination of two or more thereof.

1-6. Polysaccharide fatty acid ester

The aqueous cosmetic preparation of the invention may contain polysaccharide fatty acid ester. Generally, in the emulsification/dispersion like the aqueous cosmetic preparation of the invention, respective components having various functions may be added as forms of organic or inorganic salts in order to add the components stably. Thus, when organic salts or inorganic salts are increased, phenomena such as white turbidity, aggregation, precipitation, thickening, or separation: so-called salting out tends to occur easily due to the salts. Particularly, the transparency may be impaired by the salting out when the formulation emphasizes the transparency. When the aqueous cosmetic preparation of the invention contains polysaccharide fatty acid ester, in addition to dispersion stability, the so-called salting out may be well suppressed even if various organic or inorganic salts are added.

Further, the temporal stability of the aqueous cosmetic preparation as the suppression of precipitation of the ceramide dispersion may be improved.

In the polysaccharide fatty acid ester, a polysaccharide portion is constituted by the sugar unit such as glucose or fructose and has an average degree of polymerization of 2 to 140. Examples thereof include disaccharides such as sucrose; and polysaccharides larger than hexasaccharides such as oligosaccharide, inulin (2 to 60 fructose-units are linearly linked to a glucose moleculose), starch or dextrin. From a viewpoint of inhibiting effect of salting out phenomena (i.e., white turbidity, aggregation, precipitation, thickening, and separation) in the aqueous cosmetic preparation caused by addition of salt, dextrin, inulin or combinations thereof are preferable, and inulin is further preferable. Inulin is an oligosaccharide that contains D-fructose as a basic component, and the furanoid fructose unit with a structure having $\beta$-1,2-linked furanoid fructose and $\alpha$-D-glucose linked to sucrose at the reducing end is generally about from 2 to 60.

A fatty acid portion which forms ester with the polysaccharide is preferably a fatty acid portion having 12 to 18 carbon atoms from a viewpoint of inhibiting effect of salting out. Examples the fatty acid include caprylic acid, capric acid, lauric acid, myristic acid, palmitic acid, oleic acid, stearic acid, isostearic acid, linolic acid, linolenic acid, ethylhexanoic acid, behenic acid, and behenic acid.

Examples of the polysaccharide fatty acid ester include a dextrin fatty acid ester, a sucrose fatty acid ester, a starch fatty acid ester, an oligosaccharide fatty acid ester, and an inulin fatty acid ester. The inulin fatty acid ester and the dextrin fatty acid ester are preferable. Examples of the inulin fatty acid ester include inulin octanoate, inulin decanoate, inulin laurate, inulin myristate, Inulin lauryl carbamate, inulin palmitate, inulin stearate, inulin arachidate, inulin behenate, inulin oleate, inulin 2-ethylhexanoate, inulin isomyristate, inulin isopalmitate, inulin isostearate, and inulin isooleate. From a viewpoint of stability of the aqueous cosmetic preparation, inulin stearate, lauryl carbamate, dextrin palmitate, dextrin palmitate/octanoate, and dextrin myristate are preferable as the polysaccharide fatty acid ester of the invention. Inulin lauryl carbamate is the most preferable.

Inulin lauryl carbamate has an HLB value of about 8 and a low solubility in oily components, however, it has a good dispersibility, a low solubility in water, and aggregates in the aqueous phase. When the inulin skeleton is hydrated in an oil-in-water type emulsion, inulin lauryl carbamate is located on the surface of dispersed particles and a three-dimensional barrier is formed. Then, an emulsification structure in which the disperse particles are surrounded in the aqueous phase by the three-dimensional barrier is formed.

The polysaccharide fatty acid ester may be used alone or in combination of two or more thereof. The amount of the polysaccharide fatty acid ester may be from 0.1 to 2 times of the amount of the ceramide analog in the ceramide dispersion. From a viewpoint of stability of the aqueous cosmetic preparation, the amount is further preferably from 0.5 to 1.5 times. The content of the polysaccharide fatty acid ester is preferably from 0.05% by mass to 2% by mass, further preferably from 0.5% to 1.5% by mass relative to the total mass of the ceramide dispersion. When the content of the polysaccharide fatty acid ester is 0.05% by mass or more, it is preferable in that sufficient effects may be expected. When the content of the polysaccharide fatty acid ester is 2% by mass or less, it is preferable in that the ceramide dispersion may be maintained at an appropriate viscosity.

The polysaccharide fatty acid ester may be added to either the aqueous phase or the oil phase, which may be suitably selected according to the selected type of the polysaccharide fatty acid ester. For example, inulin lauryl carbamate may be contained as the aqueous phase component in the dispersion, and dextrin palmitate may be contained as the oil phase component in the dispersion.

1-7. Water-soluble organic solvent

The aqueous cosmetic preparation of the invention may contain the water-soluble organic solvent. In this regard,

the water-soluble organic solvent is not included in the "oil component" in the present specification.

The water-soluble organic solvent in the invention is used for mixing with an aqueous solution to be described later as an oil phase containing a natural component. At the same time, the water-soluble organic solvent is a main component of the extract which extracts the natural component. That is, in the invention, the natural component is used in a state where it is dissolved in the extract containing the water-soluble organic solvent as the main component and mixed with the aqueous solution.

The term "water-soluble organic solvent" to be used in the invention means an organic solvent whose solubility to water (at 25°C) is 10% by mass or more. The solubility to water is preferably 30% by mass or more, further preferably 50% by mass or more from the viewpoint of the stability of the produced dispersion.

The water-soluble organic solvent may be used alone or a mixture solvent of a plurality of the water-soluble organic solvents may be used. Further, it may be used as a mixed with water. When the mixture with water is used, the content of the water-soluble organic solvent is preferably 50% by volume or more, more preferably 70% by volume or more.

The water-soluble organic solvent is preferably used to mix the oil phase component and prepare the oil phase when the ceramide dispersion is prepared in the method for producing the aqueous cosmetic preparation to de described later. It is preferable that the water-soluble organic solvent is removed after it is mixed with the aqueous phase.

Examples of the water-soluble organic solvent include methanol, ethanol, 1-propanol 2-propanol, 2-butanol, acetone, tetrahydrofuran, acetonitrile, methyl ethyl ketone, dipropylene glycol monomethyl ether, methyl acetate, methyl acetoacetate, N-methylpyrrolidone, dimethylsulfoxide, ethylene glycol, 1,3-butanediol, 1,4-butanediol, propylene glycol, diethylene glycol, triethylene glycol, and mixtures thereof. Among them, ethanol, propylene glycol or acetone is preferable and a mixed solution of ethanol and water is particularly preferable when their applications are limited to food products.

1-8. Other components

In addition to the components, other additives (for example, various medicinal components, antiseptic agents, and coloring agents) which are generally used for the application may be used together, depending on the application of the aqueous cosmetic preparation of the invention unless the effect of the invention is impaired.

Examples of the other additives include a moisturizing agent such as glycine betaine, xylitol, trehalose, urea, neutral amino acid or basic amino acid; a drug efficacy agent such as allantoin; an organic powder such as cellulose powder, nylon powder, crosslinked silicone powder, crosslinked methylpolysiloxane, porous cellulose powder and porous nylon powder; an inorganic powder such as anhydrous silica, zinc oxide, and titanium oxide; a refreshing agent such as menthol and camphor, a plant extract, a pH buffer, an antioxidant, an ultraviolet absorbing agent, an ultraviolet scattering agent, an antiseptic, a perfume, a fungicide, and a coloring matter.

In the aqueous cosmetic preparation of the invention, when a ceramide analog-containing particle is used for the oil phase together with another oil component, the particle diameter of the dispersed particle contained as the oil phase may be made smaller by factors such as stirring conditions (shearing force, temperature, and pressure) in the method for producing the ceramide dispersion described below, conditions of use of the micro mixer, or the ratio of the oil phase to the aqueous phase, in addition to factors caused by the components contained in the aqueous cosmetic preparation, so that fine-grained oil-phase particles with a particle diameter of 150 nm or less may be obtained.

The transparency of the aqueous cosmetic preparation of the invention may be roughly determined by visually confirming the appearance. Generally, it may be determined by the turbidity of the aqueous cosmetic preparation. The turbidity of the aqueous cosmetic preparation may be measured as an absorbance of 660 nm at 25°C in a cell of 10 mm using UV-VIBLE spectral photometer UV-2550 (manufactured by Shimadzu Corporation). When the turbidity of the aqueous cosmetic preparation of the invention is measured using an absorbance of 660 nm and the value is 0.050 or less, the aqueous cosmetic preparation is evaluated as a transparent aqueous cosmetic preparation. The transparency of the aqueous cosmetic preparation is preferably 0.040 or less.

The pH of the aqueous cosmetic preparation of the invention is preferably from 5 to 9, more preferably from 6 to 8.5. When the pH of the aqueous cosmetic preparation is within the range, the aqueous cosmetic preparation having good dispersion stability and preservation stability is obtained. Various pH regulating agents may be used in order to adjust the pH of the aqueous cosmetic preparation to the range.

In the production process of the aqueous cosmetic preparation, the pH regulating agent may be added or blended so as to have a pH within a predetermined range of pH values when the oil phase or aqueous phase is prepared or may be directly added to the obtained aqueous cosmetic preparation. Usable examples of the pH regulating agent include an acid such as hydrochloric acid or phosphoric acid; an alkali such as sodium hydroxide; various inorganic salts that are generally used in the field; and a buffer such as lactic acid-sodium lactate, citric acid-sodium citrate and succinic acid-sodium succinate.

2. Method for producing aqueous cosmetic preparation

The aqueous cosmetic preparation of the invention may be produced by any method as long as it contains the fatty acid or salt thereof, the ceramide analog-containing particle dispersed in an aqueous phase as an oil phase component, and an aqueous phase component containing at least a natural polysaccharide.
One preferable method for producing the aqueous cosmetic preparation of the invention is, from the viewpoint of forming a ceramide analog-containing particle which exhibits minute size and has a good dispersion stability, a method in which a ceramide dispersion containing a fatty acid or salt thereof and a ceramide analog-containing particle dispersed in an aqueous phase as an oil phase component are prepared in advance, and then the ceramide dispersion is mixed with an aqueous composition containing other essential ingredients or optional components.
When this method is employed, an aqueous solution which contains an aqueous vehicle such as water as a main component may be used as the aqueous composition. The natural polysaccharides and polyhydric alcohol may be included in aqueous composition. The aqueous composition may be suitably selected in relation to the aqueous phase component of the ceramide dispersion.
The blending ratio of the ceramide dispersion and the aqueous composition may be any blending ratio as long as the content of each component described above is within the range of their content in the aqueous cosmetic preparation. In general, the blending ratio is preferably from 1:0.1 to 1:10000, and further preferably from 1:0.1 to 1:1000.
Hereinafter, the ceramide dispersion which may be used in a preferable method for producing the aqueous cosmetic preparation of the invention will be further described in detail.

(Ceramide dispersion)

The ceramide dispersion prepared when producing the aqueous cosmetic preparation is a transparent ceramide dispersion which includes a ceramide analog-containing particle dispersed in an aqueous phase as an oil phase component and a fatty acid component which is an oil phase component or an aqueous phase component. The ceramide dispersion may be obtained by a production method including a process of mixing an oil phase component which includes at least a ceramide analog, and an aqueous phase component, at 40°C or less.
According to the method, the oil phase component and the aqueous phase component are mixed at 40°C or less, whereby the oil phase component is well dissolved and a ceramide dispersion having excellent temporal stability and preservation stability may be obtained.
When the oil phase is prepared, it is preferable to use a water-soluble organic solvent in order to dissolve the ceramide analog. Examples of the water-soluble organic solvent to be used for this purpose may include the above described examples.
In the mixing of the aqueous phase component and the oil phase component, known methods such as a high-pressure emulsification method that applies a shearing force of 100 MPa or more or a jet injection method that directly injects the oil phase component into the aqueous phase component may be used. It is preferable to apply a method using a micro mixer in which the oil phase component and the aqueous phase component are independently passed through a micro path in which the cross-section area of the narrowest portion is 1 $\mu m^2$ to 1 $mm^2$, and then respective phases are mixed from a viewpoint of the particle diameter of the ceramide analog-containing particle, the dispersion stability, and the preservation stability.
In the mixing, it is preferable that the viscosity of the aqueous phase is 30 mPa·s or less from a viewpoint of finely-dividing of the ceramide analog-containing particle.
When the ceramide dispersion is prepared, the temperature at the time of mixing the oil phase component with the aqueous phase component is preferably 40°C or less. The temperature of 40°C or less at the time of mixing may be achieved when the oil phase component is mixed with the aqueous phase component, and the period to be set the temperature may be suitably changed depending on the method of mixing (emulsifying) to be used. In the method using the micro mixer, at least the temperature in the period immediately before mixing and immediately after dispersion may be set to 40°C or less.
Examples of the method for producing the ceramide dispersion includes:

a) preparing an aqueous phase using the aqueous vehicle (water etc.) containing a fatty acid salt (when it is existent);
b) preparing an oil phase using the oil phase component which includes at least a ceramide analog; and
c) mixing and dispersing the oil phase and the aqueous phase by the method described later, using the micro mixer to produce a ceramide dispersion (emulsion) which contains a particle containing a ceramide analog-containing particle (a dispersed particle) having a volume average particle diameter of from 1 nm to 100 nm.

The ratio (mass) of the oil phase and the aqueous phase in the emulsification dispersion is not particularly limited. The oil phase/aqueous phase ratio (mass %) is preferably from 0.1/99.9 to 50/50, more preferably from 0.5/99.5

to 30/70, further preferably from 1/99 to 20/80.

When the oil phase/aqueous phase ratio is within the above range, it is preferable in that an active component is sufficiently contained, and practically sufficient emulsion stability is obtained.

When a composition in powder form is produced using the ceramide dispersion, the composition in powder form may be obtained by adding the process of drying the ceramide dispersion in emulsion form by spray drying and the like.

In the method for producing the ceramide dispersion, the components contained in the oil phase and the aqueous phase is the same as the components of the ceramide dispersion of the invention, and a preferable example and an addition amount thereof are the same as those of the ceramide dispersion, and the preferable combination of the components is also the same.

(Micro mixer)

In the production method to be applied to the production of the ceramide dispersion, it is preferable to take a method of passing the oil phase component and the aqueous phase component each independently through a micro path in which the cross-section area of the narrowest portion is from 1 $\mu m^2$ to 1 $mm^2$, and combining and mixing respective components in order to stably form a ceramide analog-containing particle having a volume average particle diameter of from 1 nm to 100 nm.

The mixing of the oil phase component and the aqueous phase component is preferably mixing by countercurrent collision from a viewpoint of obtaining the nner dispersed particle.

The most suitable device for mixing by countercurrent collision is a countercurrent collision-type micro mixer. The micro mixer mixes mainly two different liquids in a fine space, one of liquids is an organic solvent phase containing a functional oil component, and the other is an aqueous phase which is an aqueous solution.

When the micro mixer is applied to preparation of an emulsion having the small particle diameter which is one of microchemistry processes, a good emulsion or dispersion having relatively low energy and small heat production, having the more uniform particle diameter as compared with a normal stirring emulsification dispersing system or high pressure homogenizer emulsification dispersing, and also having the excellent storage stability is easily obtained. This is an optimal method for emulsifying a natural component which is easily thermally degraded.

A summary of a method of emulsification or dispersing using the micro mixer include dividing the aqueous phase and the oil phase into fine spaces, respectively, and contacting or colliding respective fine spaces. This method is clearly different from a membrane emulsification method or a micro channel emulsification method which is a method in which only one is divided into a fine space, and the other is a bulk and, even when only one is actually divided into a fine space, the effect as in the invention is not obtained. As the known micro mixer, there are a variety of structures. When attention is paid to flow and mixing in a micro path, there are two kinds of a method of mixing while a laminar flow is maintained, and a method of mixing while disturbed, that is, in a disturbed flow. In the method of mixing while a laminar flow is maintained, mixing is effectively performed by making a size of a path depth greater than a path width, thereby, increasing the area of an interface between two liquids as much as possible, and making thicknesses of both layers smaller. Alternatively, a method of adopting a multilayer flow by dividing an entrance for two liquids into many potions, and flowing two liquids alternately has been also devised.

On the other hand, in a method of mixing with the disturbed flow, a method of flowing respective flows at a relatively high speed by dividing them into narrow paths is general. A method of ejecting one of fluids into the other liquid introduced into a fine space using an arrayed micro-nozzle has been also proposed. Alternatively, a method of forcibly contacting liquids flowing at a high speed using various means is good, particularly in the mixing effect. In the former method using a laminar flow, generally, a produced particle is large, and distribution is relatively uniform, on the other hand in the latter method using a disturbed flow, there is a possibility that a very fine emulsion is obtained. In respect of stability and transparency, the method using a disturbed flow is preferable in many cases. As the method using a disturbed flow, a comb tooth type and a collision type are representative. The comb tooth type micro mixer has a structure in which two comb tooth-like paths are faced, and arranged so that one path enters between two the other paths, alternately a representative of which is a mixer manufactured by IMM.

The collision-type micro mixer, represented by a KM mixer, has a structure in which forcible contact is tried utilizing the kinetic energy. Specifically, there is a central collision-type micro mixer disclosed by Nagasawa et al. ("H.Nagasawa et al., Chem.Eng.Technol.,28,No.3,324-330 (2005)", JP-A No.2005-288254). In the method of countercurrently colliding an aqueous phase and an organic solvent phase, since a mixing time is extremely short, and an oil phase droplet is instantly formed, an extremely fine emulsion or dispersion is easily formed.

In the invention, when emulsification is performed by micro-mixing with the collision-type micro mixer, a temperature at emulsification (emulsification temperature) is such that micro-mixing is performed at a temperature of the aforementioned separate fine space of the micro mixer (temperature at micro-mixing part of micro mixer) at preferably 40°C or lower, more preferably 0°C to 40°C, particularly preferably 5°C to 30°C, from a viewpoint of particle diameter

uniformity of the resulting emulsion. By adopting the emulsification temperature of 0°C or higher, since a main component of a dispersing medium is water, the emulsification temperature may be managed, being preferable. A retained temperature of the fine space of the micromixer is preferably 40°C or lower. By adopting the retained temperature of 40°C or lower, management of the retained temperature may be easily controlled, and the micro-bumping phenomenon which adversely influences on emulsification performance may be excluded. It is further preferable that the retained temperature is controlled at a temperature of 35°C or lower.

In the invention, it is particularly preferable that retained temperatures of the aqueous phase and the oil phase before and after division into the fine space of the micro mixer, and of the fine space of the micro mixer and the separate fine space are higher than room temperature and, after micro-mixing and emulsification, an oil-in-water emulsion obtained with the micro mixer is cooled to a normal temperature after collection.

The cross-sectional area of a narrowest part of the fine space (path) of the micro mixer in the invention is 1 $\mu m^2$ to 1 $mm^2$ and, from a viewpoint of miniaturization of the emulsion particle diameter and sharpness of the particle diameter distribution, preferably 500 $\mu m^2$ to 50,000 $\mu m^2$.

The cross-sectional area of a narrowest part of the fine space (path) of the micro mixer used in the aqueous phase in the invention is particularly preferably 1,000 $\mu m^2$ to 50,000 $\mu m^2$ from a viewpoint of mixing stability.

The cross-sectional area of a narrowest portion of the fine space (path) of the micro mixer used in the oil phase is particularly preferably 500 $\mu m^2$ to 20,000 $\mu m^2$ from a viewpoint of miniaturization of the emulsion particle diameter and sharpness of the particle diameter distribution.

When emulsification and dispersing are performed with the micro mixer, the flow rate of the oil phase and the aqueous phase at emulsification and dispersing are different depending on the micro mixer used and, from a viewpoint of miniaturization of the emulsion particle diameter and sharpness of the particle diameter distribution, the flow rate of the aqueous phase is preferably 10 ml/min to 500 ml/min, more preferably 20 ml/min to 350 ml/min, particularly preferably 50 ml/min to 200 ml/min.

The flow rate of the oil phase, from a viewpoint of miniaturization of the emulsion particle diameter and sharpness of the particle diameter distribution, is preferably 1 ml/min to 100 ml/min, more preferably 3 ml/min to 50 ml/min, particularly preferably 5 ml/min to 50 ml/min.

The value obtained by dividing flow rates of both phases by the cross-sectional area of a micro channel, that is, the flow speed ratio (Vo/Vw) of both phases is preferably in the range from 0.05 to 5 from a viewpoint of miniaturization of a particle and design of the micromixer, wherein Vo is the flow speed of an organic solvent phase containing a water-insoluble natural component, and Vw is the flow speed of an aqueous phase. And, the flow speed ratio (Vo/Vw) from 0.1 to 3 is the most preferable range from a viewpoint of further miniaturization of a particle.

In addition, liquid sending pressures of the aqueous phase and the oil phase are preferably 0.030 MPa to 5 MPa and 0.010 MPa to 1 MPa, more preferably 0.1 MPa to 2 MPa and 0.02 MPa to 0.5 MPa, particularly preferably 0.2 MPa to 1 MPa and 0.04 MPa to 0.2 MPa, respectively. By adopting the liquid sending pressure of the aqueous phase of 0.030 MPa to 5 MPa, it is preferable in that the stable solution sending flow rate tends to be maintained. By adopting the liquid sending pressure of the oil phase of 0.010 MPa to 1 MPa, it is preferable in that the uniform mixing property tends to be obtained.

In the invention, the flow rate, the solution sending pressure and the retained temperature are more preferably a combination of respective preferably examples.

Then, a route from introduction of the aqueous phase and the oil phase into the micro mixer to discharge as an O/W emulsion will be explained using an example of a micro device (Fig. 1) as one example of the micro mixer in the invention.

As shown in Fig. 1, a micro device 100 is constructed of a supply element 102, a confluence element 104 and a discharge element 106, each in a cylindrical form.

On a surface opposite to the confluence element 104 of the supply element 102, a cross-section as a path for the oil phase or the aqueous phase in the invention is such that rectangular annular channels 108 and 110 are concentrically formed. In the supply element 102, bores 112 and 114 leading to each annular channel are formed, penetrating in a direction of its thickness (or height) direction.

In the confluence element 104, a bore 116 penetrating in its thickness direction is formed. In this bore 116, when an element is secured thereto in order to construct the micro device 100, an end 120 of the bore 116 situated on a surface of the confluence element 104 opposite to the supply element 102 is opened in the annular channel 108. In an embodiment shown, four bores 116 are formed, and they are arranged at an equal interval in a circumferential direction of the annular channel 108.

In the confluence element 104, a bore 118 is formed, penetrating therethrough, like the bore 116. The bore 118 is formed so as to be opened in the annular channel 110, like the bore 116. Bores 118 are arranged at an equal interval in a circumferential direction of the annular channel 110, and the bore 116 and the bore 118 are arranged so as to be positioned alternately.

On a surface 122 opposite to the discharge element 106 of the confluent element 104, the micro channels 124

and 126 are formed. One end of this micro channel 124 or 126 is an opening part of the bore 116 or 118, the other end is a center 128 of the surface 122, and all micro channels extend from bores towards this center 128, and are converged at a center. A cross-section of the micro channel may be, for example, rectangular.

In the discharge element 106, a bore 130 passing a center thereof and penetrating in a thickness direction is formed. Therefore, this bore is opened in the center 128 of the confluence element 104 at one end, and is opened in the outside of the micro device at the other end.

In the present micro device 100, fluids A and B supplied from the outside of the micro device 100 at ends of bores 112 and 114 are flown into annular channels 108 and 110 via bores 112 and 114, respectively.

The annular channel 108 and the bore 116 are communicated, and the fluid A which has flown into the annular channel 108 enters a micro channel 124 via the bore 116. In addition, the annular channel 110 and the bore 118 are communicated, and the fluid B which has flown into the annular channel 110 enters a micro channel 126 via the bore 118. Fluids A and B are flown into micro channels 124 and 126, respectively, and are flown towards a center 128, and are converged.

The converged fluids are discharged as a stream C to the outside of the micro device via the bore 130.

Such the micro device 100 may have the following specifications. Cross-sectional shape of annular channel 108/width/depth/diameter : rectangle/1.5/1.5/25 mm Cross-sectional shape of annular channel 110/width/depth/diameter : rectangle/1.5/1.5/20 mm Diameter and length of bore 112: 1.5/10 mm (circular cross-section)
Diameter and length of bore 114: 1.5/10 mm (circular cross-section)
Diameter and length of bore 116: 0.5/4 mm (circular cross-section)
Diameter and length of bore 118: 0.5/4 mm (circular cross-section) Cross-sectional shape of micro channel 124/width/depth/length/ cross-sectional area: rectangle/ 350 $\mu$m/100 $\mu$m/12.5 mm/35000 $\mu m^2$
Cross-sectional shape of micro channel 126/ width/depth/length/ cross-sectional area: rectangle/50 $\mu$m/100 $\mu$m/10 mm/5000 $\mu m^2$
Diameter and length of pore 130 : 500 $\mu$m/10 mm (circular cross-section)

The size of the micro channel (in Fig. 1, 124 and 126) in which the aqueous phase and the oil phase are collided is defined in the preferable range in context with flow rates of the aqueous phase and the oil phase.

In the invention, the micro mixer disclose in JP-ANo.2004-33901 may be also preferably used.

Fig. 2 is a schematic cross-sectional view of T-type microreactor, showing one example of a mixing mechanism with a T-type microreactor. Fig. 3 is a conceptional view of a T-type microreactor, showing one example of a mixing mechanism with a T-type microreactor.

In Fig. 2, a cross-section of a T-type path 200 of a T-type microreactor is shown. In the T-type path 200, a fluid which has been flown therein in a direction of an arrow D through an inlet 202a, and a fluid which has been flown therein in a direction of an arrow E through an inlet 202b are collided at a central part in a path of the T-type path 200, and mixed to become a fine fluid particle. The fine fluid particle is flown out in a direction of an arrow F through an outlet 204. This T-type microreactor is useful for mixing when the volume of a path is small.

In Fig. 3, a fluid mixing mechanism (concept) 300 of other T-type microreactor is shown. In the fluid mixing mechanism shown in Fig. 3, fluids which have been flown therein through two paths 302a and 302b are mutually collided and mixed to become a fine fluid particle. That is, the fluid, on one hand, is flown in a path 302a in a direction of an arrow G, and is flown out in a direction of an arrow H. On the other hand, the fluid is flown in a path 302b in a direction of an arrow I, and is flown out in a direction of an arrow J. Fluids which have been flown out through paths 302a and 302b, respectively, are collided, are mixed, and are flied approximately orthogonal with a direction of an arrow G to J. The fluid mixing mechanism described in the path figure, Fig. 3, collides and mixes fluids diffused by a procedure of misting. By this collision and mixing, the fluid becomes finer, and a great contact surface may be obtained.

In the production method which may be applied to the method for producing the ceramide dispersion, it is preferable that a water-soluble organic solvent which has been used is removed after emulsification and dispersing through the micropath. As a method of removing a solvent, an evaporation method using a rotary evaporator, a flash evaporator, or an ultrasound atomizer, and a membrane separating method such as an ultrafiltration membrane and a reverse osmosis membrane are known, and an ultrafiltration membrane method is particularly preferable.

An ultra filter (abbreviated as UF) is an apparatus by which a stock solution (water, mixed aqueous solution of high-molecular substance, low-molecular substance, and colloidal substance) is pressurized, and water is poured into a UF apparatus, thereby, the stock solution may be separated into two-system solutions of a permeated solution (low-molecular substance) and a concentrated solution (high-molecular substance, colloidal substance), and taken out.

The ultrafiltration membrane is a typical asymmetric membrane made by the Leob-Sourirajan method. A polymer material used includes polyacrylonitrile, polyvinyl chloride-polyacrylonitrile copolymer, polysulfone, polyether sulfone, vinylidene fluoride, aromatic polyamide, and cellulose acetate. Recently, a ceramic membrane has become to be used. Unlike a reverse osmosis method, in an ultrafiltration method, since pre-treatment is not performed, fouling occurs, in which a polymer is deposited on a membrane surface. For this reason, it is normal to wash the membrane with a chemical or warm water periodically. For this reason, a membrane material is required to have resistance to a chemical and heat

resistance. As a membrane module of an ultrafiltration membrane, there are various kinds such as flat membrane type, tubular type, hollow thread type, and spiral type. An index for performance of an ultrafiltration membrane is a fractionation molecular weight, and various membranes having a fractionation molecular weight of 1,000 to 300,000 are commercially available. As the commercially available membrane module, there are Microsa UF (Asahi Kasei Chemicals Corporation), and capillary-type element (trade name: NTU-3306, manufactured by Nitto Denko Corporation), being not limiting.

For removing a solvent from the obtained emulsion, a material of a membrane is particularly preferably polysulfone, polyether sulfone, and aromatic polyamide are particularly preferable from a viewpoint of solvent resistance. As the form of a membrane module, a flat membrane is mainly used at a laboratory scale, and a hollow shred type and spiral type are industrially used, and a hollow shred type is particularly preferable. In addition, a fraction molecular weight is different depending on a kind of an active ingredient and, usually, the range of 5,000 to 100,000 is used.

An operation temperature may be 0°C to 80°C and, in view of degradation of an active ingredient, the range of 10°C to 40°C is particularly preferable.

As an ultrafiltration device at a laboratory scale, there are ADVANTEC-UHP (ADVANTEC), Flow Type Labotest Unit RUM-2 (Nitto Denko Corporation) using and a flat membrane-typed module. Industrially, respective membrane modules at the size and the number depending on the necessary potency may be arbitrarily combined to construct a plant. As a bench scale unit, RUW-5A (Nitto Denko Corporation) is commercially available.

In the production method which may be applied to the method for producing the ceramide dispersion, a process of concentrating the resulting emulsion subsequent to solvent removal may be added. As the concentrating method, the same method and the device as those of solvent removal such as an evaporation method and a filtration membrane method may be used. Also in the case of concentration, an ultrafiltration membrane method is a preferable method. When the same membrane as that of solvent removal may be used, this is preferable and, if necessary, ultrafiltration membranes having different fractionation molecular weights may be also used. Alternatively, a concentration efficacy may be enhanced by operating at a temperature different from that of solvent removal.

The ceramide dispersion obtained by mixing with the micro mixer is an O/W emulsion. In the invention, the volume average particle diameter (median diameter) of the ceramide analog-containing particle contained in the ceramide dispersion is from 2 nm to 150 nm. From a viewpoint of transparency of the resulting dispersion, the diameter is more preferably 5 nm to 50 nm. The particle diameter of the ceramide analog-containing particle (dispersed particle) may be measured with a commercially available particle size distribution meter, and details thereof are as described above.

3. Application of aqueous cosmetic preparation

The cosmetic preparation of the invention may be any formulation of face lotion, essence, gel, milky lotion, cream, and facial wash which are general formulations known as cosmetic preparations. In order to make full use of the characteristics of the smallness of the particle diameter of the ceramide analog-containing particle in the invention, it is preferable to employ a highly transparent formulation. Particularly, face lotion, essence, and gel preparations are preferable.

The disclosure of Japanese Patent Application No. 2008-254538 is incorporated herein by reference in its entirety. All references, patent applications, and technical standards described in the present specification are herein incorporated in their entirety by reference into the specification, to the same extent as if each individual reference, patent application or technical standard was specifically and individually indicated to be incorporated herein by reference.

In the present specification, ranges indicated with "to" mean ranges including the numerical values before and after "to" as the minimum and maximum values.

EXAMPLES

The present invention will be further specifically explained below by way of Examples, but the invention is not limited to the following Examples as far as it is not departed from the gist thereof. Unless otherwise is indicated, "part" is on a mass basis.

(Preparation of ceramide dispersion -1)

Respective components described in the following composition of oil phase liquid 1 was stirred at room temperature for about 30 minutes to prepare an oil phase liquid 1.

In the preparation of an aqueous phase liquid 1, inulin lauryl carbamate described in the following composition of aqueous phase liquid 1 was added to pure water, which was heated to about 50°C, and sufficiently stirred and dissolved. Thereafter, the remaining components were added thereto, and the liquid temperature was adjusted to 30°C.

<Composition of oil phase liquid 1>

| | |
|---|---|
| Ceramide 3B [natural ceramide, Specific example 1-10] | 0.9 part |
| Ceramide 6 [natural ceramide, Specific example 1-7] | 1.1 parts |
| Oleic acid (melting point: 14°C) | 0.4 part |
| Ethanol [water-soluble organic solvent] | 97.6 parts |

<Composition of aqueous phase liquid 1>

| | |
|---|---|
| Pure water | 96.86 parts |
| Inulin lauryl carbamate | 0.29 part |
| Glycerol | 1.43 parts |
| 1,3-butanediol | 1.43 parts |
| Sodium hydroxide | appropriate amount |

The resulting oil phase liquid 1 (oil phase) and aqueous phase liquid 1 (aqueous phase) were micro-mixed at the ratio (mass ratio) of 1:7 using a KM-type micro mixer 100/100 which is an collision type, to obtain a ceramide dispersion-1 having a liquid temperature of 30°C. The condition for using the micro mixer are as follows.

Micro channel -

Oil phase side micro channel
Cross-sectional phase/width/depth/length=rectangular/70 μm/100 μm/10 mm
Aqueous phase side micro channel
Cross-sectional phase/width/depth/length=rectangular/490 μm/100 μm/10 mm

- Flow rate -

An aqueous phase was introduced into an external annulus at the flow rate of 21.0 ml/min, an oil phase was introduced into an internal annulus at the flow rate of 3.0 ml/min, and these were micro-mixed.

The resulting ceramide dispersion liquid 1 was repeatedly desolvated to the ethanol concentration of 0.1% or less using EVAPOR (CEP-lab) manufactured by OKAWARA CORPORATION, and this was concentrated and adjusted to the ceramide concentration of 1.0% to obtain a ceramide dispersion A having a pH of 7.5. The ceramide concentration referred herein is the content of the ceramide analog based on the total mass of the ceramide dispersion.

(Preparation of ceramide dispersion -2)

Respective components described in the following composition of oil phase liquid 2 was stirred at room temperature for 1 hour to prepare an oil phase liquid 2.

A ceramide dispersion liquid 2 was obtained in the same manner as described in Preparation of ceramide dispersions -1 except that the oil phase liquid 1 was changed to the oil phase liquid 2, and the oil phase liquid 2 (oil phase) and an aqueous phase liquid 2 having the following composition were respectively heated to 40°C in Preparation of ceramide dispersions -1. Further, the obtained ceramide dispersion liquid 2 was concentrated and adjusted in the same manner as described in Preparation of ceramide dispersions -1, thereby a ceramide dispersion B having a pH of 6.2 was obtained.

<Composition of oil phase liquid 2>

| | |
|---|---|
| Ceramide 3B [natural ceramide, Specific example 1-10] | 0.9 part |
| Ceramide 6 [natural ceramide, Specific example 1-7] | 1.1 parts |
| Isostearic acid (melting point: -10°C) | 0.4 part |
| Ethanol [water-soluble organic solvent] | 76.0 parts |

<Composition of aqueous-phase solution 2>

Pure water
Sodium hydroxide appropriate amount

(Preparation of ceramide dispersions-3)

A ceramide dispersion liquid 3 was obtained in the same manner as described in Preparation of ceramide dispersions -1 except that the oil phase liquid 2 was changed to an oil phase liquid 3 having the following composition and the obtained oil phase liquid 2 (oil phase) and water (aqueous phase) were respectively heated to 30°C in Preparation of ceramide dispersions -2. Further, the obtained ceramide dispersion liquid 3 was concentrated and adjusted in the same manner as described in Preparation of ceramide dispersions -2 and a ceramide dispersion C having a pH of 7.3 was obtained.

<Composition of oil-phase solution 3>

| | |
|---|---|
| Ceramide 3B [natural ceramide, Specific examples 1-10] | 0.9 part |
| Ceramide 6 [natural ceramide, Specific examples 1-7] | 1.1 parts |
| Oleic acid (melting point: 14°C) | 0.4 part by mass |
| Ethanol [water soluble organic solvent] | 76.0 parts |

Examples 1 to 10, Comparative example 1

(Preparation of aqueous cosmetic preparation)

Respective components except the ceramide dispersion was mixed and dissolved at room temperature so that the components contained in the resultant aqueous cosmetic preparation was based on the kind and amount as described in Table 1. Thereafter, the ceramide dispersion was added so that the ceramide dispersion contained in the resultant aqueous cosmetic preparation was based on the kind and amount as described in Table 1, and the remaining amount was adjusted with water so as to be 100 parts by mass in total.

<Evaluation>

1. Measurement of pH

The pH was measured using a pH meter (F-54, manufactured by HORIBA Ltd.) for the glass electrode method. The results are shown in Table 1.

2. Measurement of electric conductivity

The electric conductivity was measured using an electric conductivity meter (F-54, manufactured by HORIBA Ltd.) for the AC two-electrode method. The results are shown in Table 1.

3. Particle diameter of ceramide analog-containing particle

The particle diameter of the ceramide analog-containing particle (or oil droplet-like dispersion particles containing the same) in respective aqueous cosmetic preparations immediately after preparation was measured using a dynamic light scattering particle size distribution meter LB-550 (manufactured by HORIBA Ltd.). In the measurement of the particle diameter, the ceramide analog-containing particles were diluted with pure water so as to have a concentration of 1% by mass, and the particle diameter was measured using quartz cell. The particle diameter was determined as a median diameter when the refractive index of a sample was 1.600, the refractive index of a dispersion medium was 1.333 (pure water), and the viscosity of pure water was set as the viscosity of the dispersion medium. The obtained particle diameter was evaluated in accordance with the following criteria. A or B is a satisfactory level from a practical viewpoint.

A: less than 30 nm
B: 30 nm or more and less than 150 nm
C: 150 nm or more

4. Evaluation of temporal stability of aqueous cosmetic preparation

Evaluation of temporal stability was performed by the following method using turbidity.

The turbidity of each aqueous cosmetic preparation of Examples 1 to 5 and Comparative Example 1 immediately after preparation was measured in terms of the absorbance of light having a wavelength of 660 nm in a cell of 10 mm using a UV-VIBLE spectral photometer UV-2550 (manufactured by Shimadzu Corporation). (Measurement temperature: 25°C.)

Further, 7 cycles (two weeks) of a step of storing each aqueous cosmetic preparation in a thermostat at 60°C for 24 hours and then storing the same in a refrigerator at 4°C for 24 hours were performed. Thereafter, the temperature was returned to 25°C and the turbidity was measured again.

For the change of turbidity of each aqueous cosmetic preparation, the difference between the turbidity after temporal storage and the turbidity immediately after preparation was calculated and evaluated in accordance with the following criteria. The results are shown in Table 1.

C: Change of turbidity is 0.1 or more (level without commercial value as a cosmetic product)
B: Change of turbidity is from 0.05 to less than 0.1 (level acceptable for commercial value as a cosmetic product).
A: Change of turbidity is less than 0.05 (level at which the change of turbidity is found; but there are no commercial value problems as a cosmetic product).

Table 1

(parts by mass)

| | Example 1 | Example 2 | Example 3 | Example 4 | Example 5 | Example 6 | Example 7 | Example 8 | Example 9 | Example 10 | Comparative example 1 |
|---|---|---|---|---|---|---|---|---|---|---|---|
| Ceramide dispersion A | 1 | 1 | 3 | 5 | 10 | | | 1 | 1 | 1 | 1 |
| Ceramide dispersion B | | | | | | 1 | | | | | |
| Ceramide dispersion C | | | | | | | 1 | | | | |
| glycerol | 3 | 3 | 3 | 3 | 3 | 3 | 3 | 3 | 3 | 3 | 3 |
| butylene glycol | 6 | 8 | 6 | 6 | 6 | 6 | 6 | 6 | 8 | 6 | 6 |
| pentylene glycol | 1.5 | 1.5 | 1 | 2 | 1.5 | 1.5 | 1 | 1.5 | 1.5 | | 1 |
| sodium chloride | | 0.1 | | | | | | 0.1 | 0.25 | 0.35 | 1 |
| ascorbyl phosphate Mg | | | 0.5 | | | | | | | | |
| citric acid | | | | appropriate amount | | | appropriate amount | | | | |
| sodium citrate | | | | | | | | | | | |
| acetylhydroxyproline | 0.01 | | | | | | | 0.01 | | | |
| methyl paraben | | 0.2 | | | | | | | 0.2 | | |
| phenoxyethanol | | | 0.2 | | | | | | | 0.2 | |
| water | remaining amount | remaining amount | remaining amount | remaining amount | remaining amount | remaining amount | remaining amount | remaining amount | remaining amount | remaining amount | remaining amount |
| electric conductivity (mS/cm) | 0.3 | 2.0 | 4.0 | 0.3 | 0.3 | 0.3 | 0.3 | 2.0 | 5.0 | 6.0 | 12.0 |
| pH | 7.0 | 7.0 | 7.0 | 6.0 | 7.0 | 7.0 | 7.0 | 4.5 | 6.0 | 7.0 | 4.0 |
| average particle diameter (nm) | A | A | A | A | A | A | A | B | B | B | C |
| temporal stability | A | A | A | A | A | A | A | B | B | B | C |

Since the aqueous cosmetic preparations of Examples 1 to 10 each contain a ceramide analog-containing particle having a fine particle diameter and exhibit little change in turbidity, it is found that these aqueous cosmetic preparations have excellent temporal stability. On the other hand, it is found that the aqueous cosmetic preparation of Comparative Example 1 has low temporal stability.

In Fig. 4, the pH of the aqueous cosmetic preparation liquids of Examples 1 to 10 and Comparative Example 1 is plotted as a horizontal axis and the electric conductivity (mS/cm) is plotted as a vertical axis. As shown in Fig. 4, it is confirmed that the pH and electric conductivity of the aqueous cosmetic preparations of Examples 1 to 10, which exhibit excellent effects, satisfy the relationship of Equation (A) (the relationship whereby electric conductivity (mS/cm) $\leq 2.2 \times$ pH-7), i.e., the suitable range of the invention.

## Claims

1. An aqueous cosmetic preparation comprising: a ceramide analog-containing particle that contains at least a ceramide analog and has a volume average particle diameter of from 2 nm to 150 nm, the particle being dispersed in an aqueous phase as an oil-phase component and the ceramide analog being selected from a group consisting of a natural ceramide, a glycosylated ceramide such as a sphingoglycolipid, a synthetic ceramide, a sphingosine and a phytosphingosine; and a fatty acid having 10 to 30 carbon atoms or a salt thereof, the aqueous cosmetic preparation satisfying a condition that the pH is 6.5 or more and a condition that the electric conductivity is 5.0 mS/cm or less.

2. The aqueous cosmetic preparation according to claim 1, wherein the pH and the electric conductivity (mS/cm) satisfy a relationship expressed by the following Equation (A):

$$\text{Electric conductivity (mS/cm)} \leq 2.2 * \text{pH-7} \qquad \text{[Equation (A)]}$$

3. The aqueous cosmetic preparation according to claim 1, wherein the volume average particle diameter of the particle is from 5 nm to 100 nm.

4. The aqueous cosmetic preparation according to claim 1, further comprising a polyhydric alcohol.

5. The aqueous cosmetic preparation according to claim 1, further comprising a macromolecular compound.

6. The aqueous cosmetic preparation according to claim 1, wherein the fatty acid having 10 to 30 carbon atoms is in liquid form at 30°C.

7. The aqueous cosmetic preparation according to claim 1, wherein the fatty acid having 10 to 30 carbon atoms or a salt thereof is at least one compound selected from the group consisting of lauric acid, isostearic acid, oleic acid, γ-linolenic acid, α-linolenic acid, and respective salts thereof.

8. A method for producing the aqueous cosmetic preparation according to claim 1 comprising:

   preparing a ceramide dispersion containing a ceramide analog containing particle, wherein the ceramide analog is selected from a group consisting of a natural ceramide, a glycosylated ceramide such as a sphingoglycolipid, a synthetic ceramide, a sphingosine, a phytosphingosine, by passing an oil phase component which contains at least a ceramide analog and an aqueous phase component which contains at least the fatty acid, through a micro path having a cross-section area at the narrowest portion thereof of from 1 $\mu m^2$ to 1 $mm^2$, whereafter combining and mixing the oil phase component and the aqueous phase component, at 40°C or less; and mixing the ceramide dispersion and an aqueous composition.

9. The method for producing the aqueous cosmetic preparation according to claim 8, further comprising dissolving the ceramide analog in a good solvent for the ceramide analog.

10. The method for producing the aqueous cosmetic preparation according to claim 9, wherein the good solvent for the ceramide analog is a water-soluble organic solvent.

## Patentansprüche

1. Wässrige kosmetische Zubereitung enthaltend: einen ein Ceramid-Analogon enthaltenden Partikel, der mindestens ein Ceramid-Analogon enthält und einen volumendurchschnittlichen Partikeldurchmesser von 2 nm bis 150 nm aufweist, wobei der Partikel in einer wässrigen Phase als eine Ölphasenkomponente dispergiert ist; und das Ceramid-Analogon ausgewählt ist aus einer Gruppe bestehend aus einem natürlichen Ceramid, einem glykosylierten Ceramid, wie einem Sphingoglykolipid, einem synthetischen Ceramid, einem Sphingosin und einem Phytosphingosin; und eine Fettsäure, die 10 bis 30 Kohlenstoffatome aufweist, oder ein Salz davon, wobei die wässrige kosmetische Zubereitung eine Bedingung, dass der pH 6,5 oder mehr beträgt und eine Bedingung, dass die elektrische Leitfähigkeit 5,0 mS/cm oder weniger beträgt, erfüllt.

2. Wässrige kosmetische Zubereitung nach Anspruch 1, wobei der pH und die elektrische Leitfähigkeit (mS/cm) eine durch die folgende Gleichung (A) ausgedrückte Beziehung erfüllen:

$$\text{elektrische Leitfähigkeit (mS/cm)} \leq 2.2 * \text{pH-7} \qquad \text{[Gleichung (A)]}$$

3. Wässrige kosmetische Zubereitung nach Anspruch 1, wobei der volumendurchschnittliche Partikeldurchmesser des Partikels 5 nm bis 100 nm beträgt.

4. Wässrige kosmetische Zubereitung nach Anspruch 1, ferner enthaltend einen mehrwertigen Alkohol.

5. Wässrige kosmetische Zubereitung nach Anspruch 1, ferner enthaltend eine makromolekulare Verbindung.

6. Wässrige kosmetische Zubereitung nach Anspruch 1, wobei die Fettsäure, die 10 bis 30 Kohlenstoffatome aufweist, bei 30 °C in flüssiger Form vorliegt.

7. Wässrige kosmetische Zubereitung nach Anspruch 1, wobei die Fettsäure, die 10 bis 30 Kohlenstoffatome aufweist, oder ein Salz davon, mindestens eine Verbindung ist, die aus der Gruppe bestehend aus Laurinsäure, Isostearinsäure, Ölsäure, $\gamma$-Linolensäure, $\alpha$-Linolensäure und entsprechenden Salzen davon ausgewählt ist.

8. Verfahren zur Herstellung der wässrigen kosmetischen Zubereitung nach Anspruch 1, umfassend:

   Herstellen einer Ceramiddispersion, die einen ein Ceramid-Analogon enthaltenden Partikel enthält, wobei das Ceramid-Analogon ausgewählt ist aus der Gruppe bestehend aus einem natürlichen Ceramid, einem glykosylierten Ceramid, wie einem Sphingoglykolipid, einem synthetischen Ceramid, einem Sphingosin, einem Phytosphingosin, durch Passieren einer Ölphasenkomponente, die mindestens ein Ceramid-Analogon enthält und einer wässrige-Phase-Komponente, die mindestens die Fettsäure enthält, durch einen Mikropfad, der an seinem engsten Bereich eine Querschnittsfläche von 1 $\mu m^2$ bis 1 $mm^2$ aufweist, hernach Vereinen und Mischen der Ölphasenkomponente und der wässrige-Phase-Komponente bei 40°C oder weniger; und Mischen der Ceramiddispersion und einer wässrigen Zubereitung.

9. Verfahren zur Herstellung der wässrigen kosmetischen Zubereitung nach Anspruch 8, ferner umfassend ein Lösen des Ceramid-Analogons in einem guten Lösungsmittel für das Ceramid-Analogon.

10. Verfahren zur Herstellung der wässrigen kosmetischen Zubereitung nach Anspruch 9, wobei das gute Lösungsmittel für das Ceramid-Analogon ein wasserlösliches organisches Lösungsmittel ist.

## Revendications

1. Préparation cosmétique aqueuse, comprenant : une particule contenant un analogue de céramide qui contient au moins un analogue de céramide et présente un diamètre de particule moyen en volume allant de 2 nm à 150 nm, la particule étant dispersée dans une phase aqueuse comme composant de phase huileuse, et l'analogue de céramide étant sélectionné parmi un groupe consistant en un céramide naturel, un céramide glycosylé tel qu'un sphingoglycolipide, un céramide synthétique, une sphingosine et une phytosphingosine, et un acide gras présentant de 10 à 30 atomes de carbone ou un sel de celui-ci, la préparation cosmétique aqueuse satisfaisant une condition

selon laquelle le pH est supérieur ou égal à 6,5 et une condition selon laquelle la conductivité électrique est inférieure ou égale à 5,0 mS/cm.

**2.** Préparation cosmétique aqueuse selon la revendication 1, dans laquelle le pH et la conductivité électrique (mS/cm) satisfont une relation exprimée par l'équation suivante (A) :

$$\text{Conductivité électrique (mS/cm)} \leq 2{,}2 * \text{pH-7} \qquad \text{[Equation (A)].}$$

**3.** Préparation cosmétique aqueuse selon la revendication 1, dans laquelle le diamètre de particule moyen en volume de la particule s'étend de 5 nm à 100 nm.

**4.** Préparation cosmétique aqueuse selon la revendication 1, comprenant en outre un polyol.

**5.** Préparation cosmétique aqueuse selon la revendication 1, comprenant en outre un composé macromoléculaire.

**6.** Préparation cosmétique aqueuse selon la revendication 1, dans laquelle l'acide gras présentant de 10 à 30 atomes de carbone est sous forme liquide à 30 °C.

**7.** Préparation cosmétique aqueuse selon la revendication 1, dans laquelle l'acide gras présentant de 10 à 30 atomes de carbone ou un sel de celui-ci est au moins un composé sélectionné parmi le groupe consistant en l'acide laurique, l'acide isostéarique, l'acide oléique, l'acide $\gamma$-linolénique, l'acide $\alpha$-linolénique, et des sels respectifs de ceux-ci.

**8.** Procédé destiné à produire la préparation cosmétique aqueuse selon la revendication 1, comprenant l'étape consistant à : préparer une dispersion de céramide contenant une particule contenant un analogue de céramide, dans lequel l'analogue de céramide est sélectionné parmi un groupe consistant en un céramide naturel, un céramide glycosylé tel qu'un sphingoglycolipide, un céramide synthétique, une sphingosine et une phytosphingosine, en faisant passer un composant à phase huileuse, qui contient au moins un analogue de céramide, et un composant à phase aqueuse, qui contient au moins l'acide gras, par un microtrajet présentant une section sur sa portion la plus étroite de 1 $\mu m^2$ à 1 $mm^2$, puis à combiner et mélanger le composant à phase huileuse et le composant à phase aqueuse à 40 °C ou moins, et à mélanger la dispersion de céramide et une composition aqueuse.

**9.** Procédé destiné à produire la préparation cosmétique aqueuse selon la revendication 8, comprenant en outre l'étape consistant à dissoudre l'analogue de céramide dans un solvant bon pour l'analogue de céramide.

**10.** Procédé destiné à produire la préparation cosmétique aqueuse selon la revendication 9, dans lequel le solvant bon pour l'analogue de céramide est un solvant organique soluble dans l'eau.

FIG.1

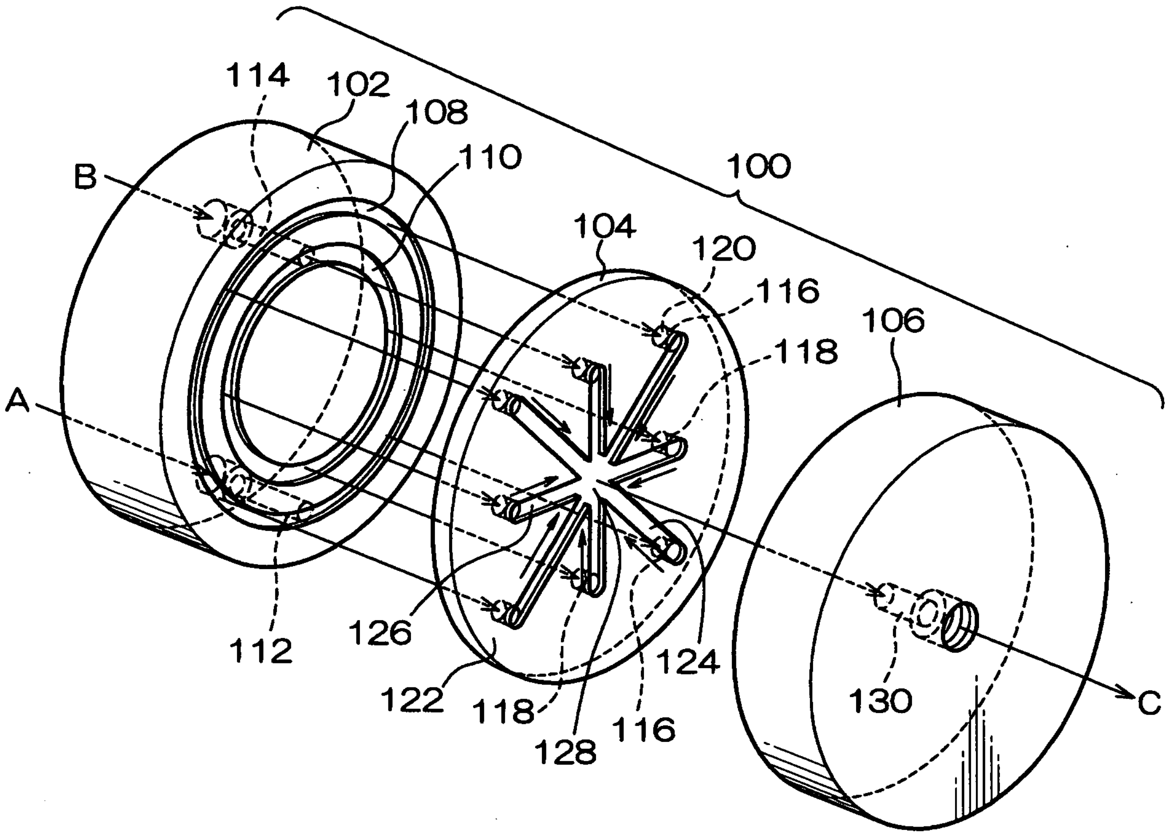
100
102
104
106
108
110
112
114
116
118
120
116
118
122
124
126
128
130
A
B
C

FIG.2

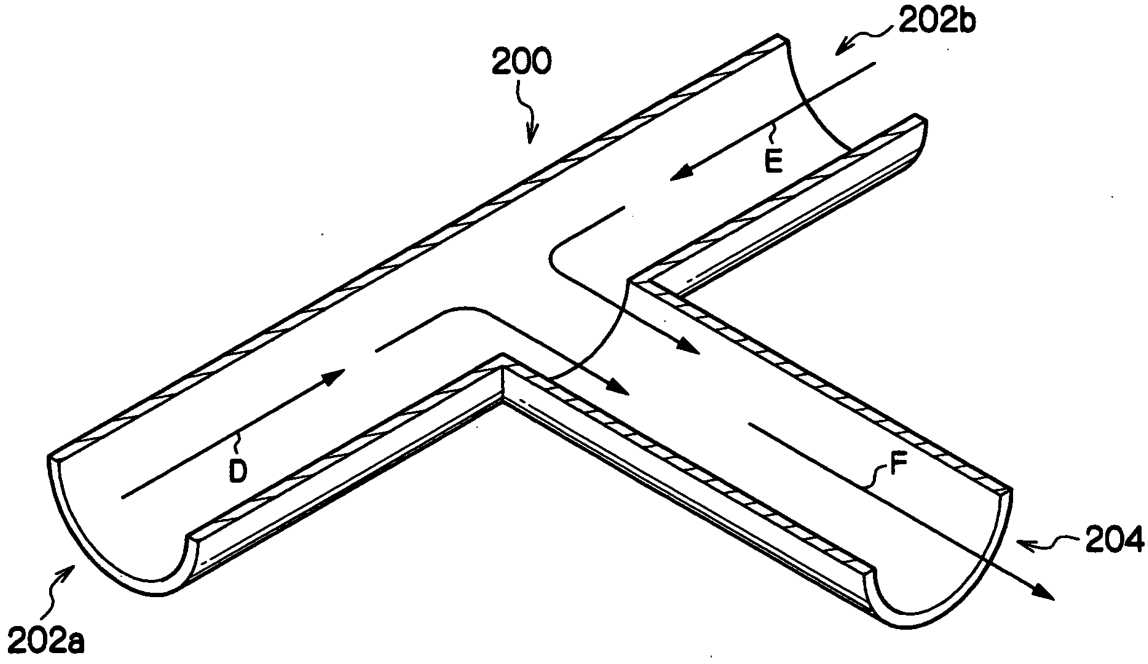
200
202a
202b
204
D
E
F

FIG.3

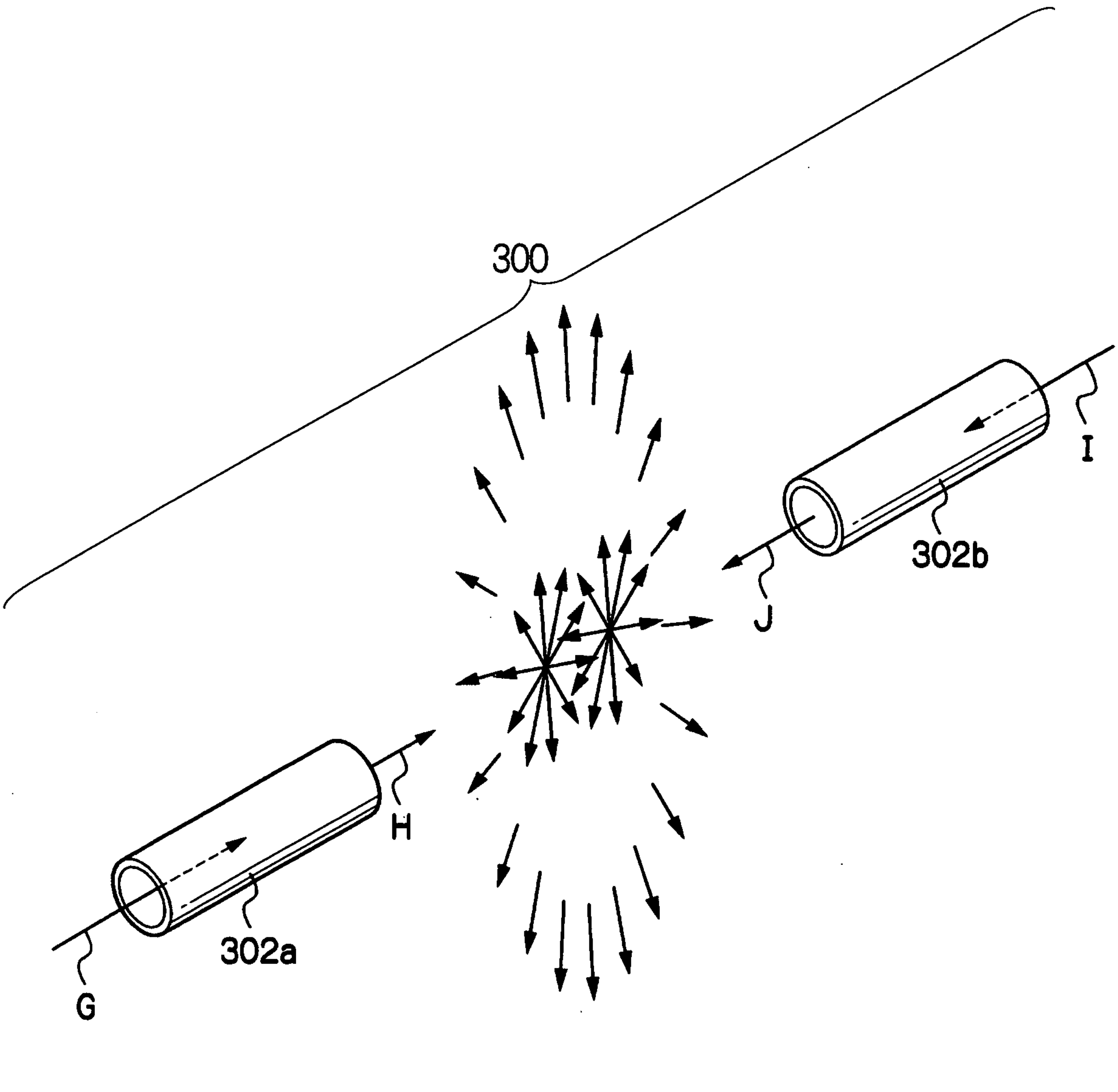

FIG.4

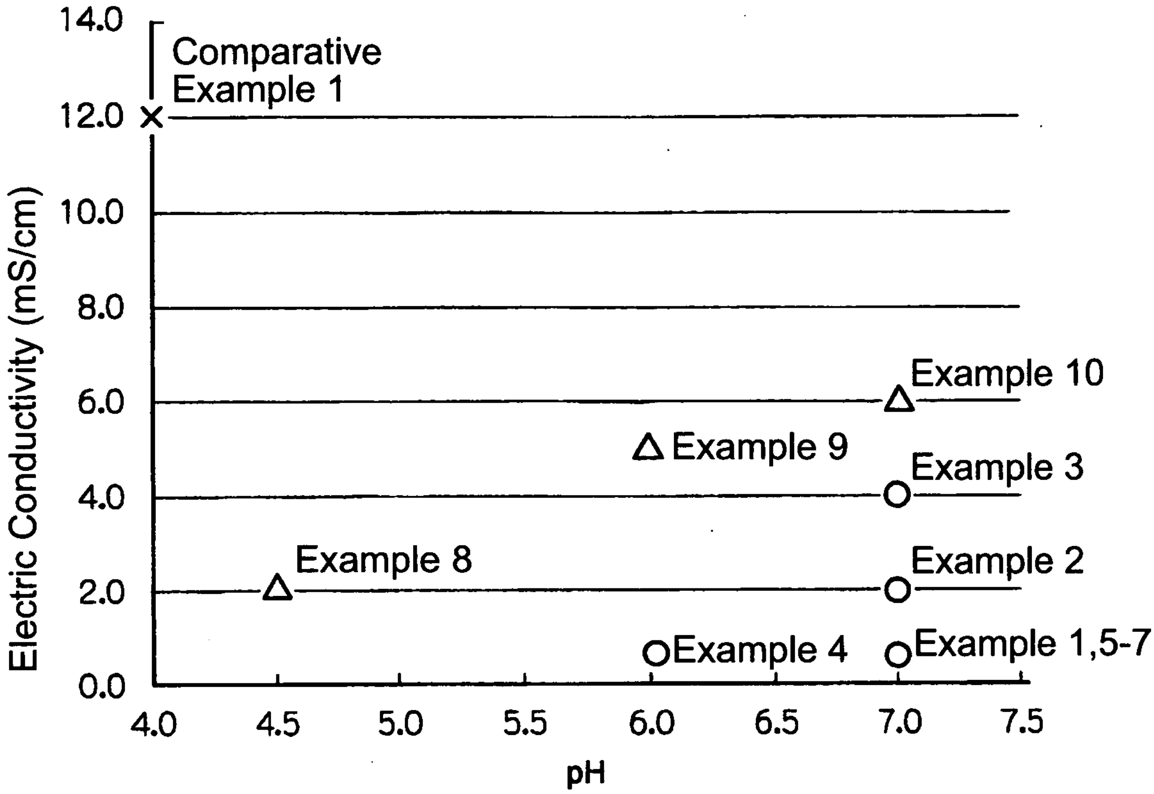
Electric Conductivity (mS/cm)
14.0
12.0
10.0
8.0
6.0
4.0
2.0
0.0
Comparative Example 1
Example 10
Example 9
Example 3
Example 8
Example 2
Example 4
Example 1,5-7
4.0
4.5
5.0
5.5
6.0
6.5
7.0
7.5
pH

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2000051676 A
- JP 7187987 A
- JP 2006335692 A
- JP 11310512 A
- JP 2001139796 A
- JP 2001316217 A
- JP 2005288254 A
- JP 2008254538 A

### Non-patent literature cited in the description

- **H. et al.** *Chem.Eng.Technol.,* 2005, vol. 28 (3), 324-330